# EUROPÄISCHE PATENTSCHRIFT

(11) **EP 3 206 720 B1**
(45) Veröffentlichungstag und Bekanntmachung des Hinweises auf die Patenterteilung: **22.04.2020**
(21) Anmeldenummer: 15781581.2
(22) Anmeldetag: 16.10.2015
(51) Int. Cl.: A61K 51/04, A61P 19/00, A61K 31/675

(54) **KONJUGIERTE BISPHOSPHONATE FÜR DIE DIAGNOSTIK UND THERAPIE VON KNOCHENERKRANKUNGEN**
CONJUGATED BISPHOSPHONATES FOR THE DIAGNOSIS AND THERAPY OF BONE DISEASES
BISPHOSPHONATE CONJUGUÉ POUR LE DIAGNOSTIC ET LE TRAITEMENT DE MALADIES OSSEUSES

(30) Priorität: 17.10.2014 DE 102014115154
(43) Veröffentlichungstag der Anmeldung: 23.08.2017
(73) Patentinhaber: SCV GmbH, 55270 Zornheim (DE)
(72) Erfinder: RÖSCH, Frank, 55126 Mainz (DE); MECKEL, Marian, 80807 Munich (DE)
(74) Vertreter: Graf von Stosch, Andreas
(86) Internationale Anmeldenummer: PCT/EP2015/002054
(87) Internationale Veröffentlichungsnummer: WO 2016/058704

(56) Entgegenhaltungen:
- EP-A1- 2 468 760
- WO-A2-02/062398
- MARIAN MECKEL ET AL: "In vivo comparison of DOTA based 68Ga-labelled bisphosphonates for bone imaging in non-tumour models", NUCLEAR MEDICINE AND BIOLOGY., Bd. 40, Nr. 6, 1. August 2013 (2013-08-01) , Seiten 823-830, XP055236221, US ISSN: 0969-8051, DOI: 10.1016/j.nucmedbio.2013.04.012 in der Anmeldung erwähnt
- KAZUMA OGAWA ET AL: "Preparation and evaluation of a radiogallium complex-conjugated bisphosphonate as a bone scintigraphy agent", NUCLEAR MEDICINE AND BIOLOGY, ELSEVIER, NY, US, Bd. 38, Nr. 5, 17. Dezember 2010 (2010-12-17), Seiten 631-636, XP028234563, ISSN: 0969-8051, DOI: 10.1016/J.NUCMEDBIO.2010.12.004 [gefunden am 2011-01-03] in der Anmeldung erwähnt
- RUSSELL R G G ET AL: "Mechanisms of action of bisphosphonates: similarities and differences and their potential influence on clinical efficacy", OSTEOPOROSIS INTERNATIONAL ; WITH OTHER METABOLIC BONE DISEASES, SPRINGER-VERLAG, LO, Bd. 19, Nr. 6, 24. Januar 2008 (2008-01-24), Seiten 733-759, XP019620436, ISSN: 1433-2965 in der Anmeldung erwähnt

## Beschreibung

Die vorliegende Erfindung betrifft eine Verbindung V zum Komplexieren von metallischen Isotopen, umfassend einen Chelator X und einen oder mehrere, mit dem Chelator X konjugierte Targetingvektoren mit der Struktur -L₁-R₁-L₂-R₂-L₃-R₃, wobei R₃ ein Bisphosphonat enthält. Im Weiteren betrifft die Erfindung ein Pharmakon, das aus der Verbindung V und einem mit der Verbindung V komplexierten, metallischen Isotop besteht, sowie ein Verfahren zur Herstellung des Pharmakons.

Bisphosphonate (BP) sind eine Stoffgruppe zur Behandlung von Erkrankungen des Knochen- und Calciumstoffwechsels. Hierzu zählen Morbus Paget, Osteoporose und die konventionelle systemische Behandlung von Knochentumoren. Bisphosphonate zeichnen sich durch eine ausgeprägte Selektivität in ihrer Anreicherung im Organismus an mineralischem Calciumphosphat aus. Im Zielgewebe lösen sie mehrere Effekte aus. Zum einen hemmen sie die Mineralisation der Knochensubstanz, zum anderen den Knochenabbau. Ihre Wirkung beruht u. a. auf der Hemmung der Farnesyl-Pyrophosphat-Synthase (FPPS), einem Enzym im HMG-CoA-Reduktase-(Mevalonat)-Weg. Durch die Hemmung des Enzyms wird die Produktion von Farnesyl, einem wichtigen Molekül zur Verankerung von Signalproteinen an der Zellmembran in der Zelle (FPPS), gehemmt. Als Folge wird die Apoptose in der Zelle eingeleitet. Dadurch haben derartige Bisphosphonat-Derivate bereits eine Therapie-relevante Funktion auch auf dem Niveau einer einzelnen Zelle.

Durch die selektive Anreicherung von Bisphosphonaten an der Knochenoberfläche betrifft die apoptotische Wirkung in besonderem Maße die osteogenen Zellen, hierbei vor allem die Osteoclasten, welche die Bisphosphonate durch die Demineralisierung der Knochenmatrix in größerem Umfang aufnehmen. Durch die Verringerung der Osteoclastenaktivität wird ein antiresorptiver Effekt erreicht.

Folgende Bisphosphonate sind heute klinisch relevant: Clodronat, Alendronat, Etidronat, Ibandronat, Risedronat und Zoledronat. Hierin werden die folgenden Abkürzungen verwendet:
ZOL = Zoledronat.
PAM = Pamidronat.

Diese Abkürzungen werden bei den hierin beschriebenen Konjugaten hochgestellt nach dem Namen oder der Abkürzung des Chelators X verwendet: beispielsweise bezeichnet DOTA^{PAM} das Pamidronat-DOTA-Konjugat.

Innerhalb der Verbindungsklasse der Bisphosphonate unterschiedet man zwischen α-H-Bisphosphonaten, α-Hydroxy-Bisphosphonaten, Stickstoff-haltigen Bisphosphonaten (N-BP) und heterozyklischen Stickstoff-haltigen Bisphosphonaten. Diese unterscheiden sich deutlich in ihrer Bindungseffektivität am Knochen, ihrer Pharmakokinetik und ihrem Inhibitionspotential der FPPS auf Grund unterschiedlicher Struktur-Wirkungsbeziehungen.

Die kinetische Bindungsaffinität zur mineralischen Knochensubstanz bzw. zu künstlichem Hydroxyapatit (HA) steigt mit folgender Reihenfolge der Verbindungen: Clodronat < Etidronat < Risedronat < Ibandronat < Alendronat < Pamidronat < Zoledronat und in der Sequenz von α-H-Bisphosphonaten zu α-Hydroxy-Bisphosphonaten. Die zusätzliche OH-Gruppe stellt eine Koordinationsstelle zur Bindung an HA dar. Ein weiterer wichtiger Faktor, der die Affinität zu HA bestimmt, ist das Stickstoff-Atom in C₃₋₄-Position zu den geminalen Bisphosphonaten der N-Bisphosphonate und heterozyklischen N-Bisphosphonate. Dieser dient ebenfalls als zusätzliche Donorstelle zu HA, hat aber auch einen entscheidenden Einfluss auf das Zeta-Potential der Bisphosphonate bei physiologischem pH-Wert. Bisphosphonate mit positiv geladener Seitenkette wie Alendronat, Pamidronat und Zoledronat zeigen höhere Bindungskapazitäten an HA (R. G. G. Russell, N. B. Watts, F. H. Ebetino, M. J. Rogers, Mechanisms of action of bisphosphonates: similarities and differences and their potential influence on clinical efficacy, Osteoporose Int. 2008; 19: 733-759).

Radioaktive Tracer auf Basis von Verbindungen, die eine Bindungskapazität zu den unterschiedlichen Funktionalitäten des HA aufweisen, werden zu diagnostischen wie auch zu therapeutischen Zwecken seit längerem routinemäßig verwendet.

Radioaktiv markierte Bisphosphonate wie [^{99m}Tc]MDP (Methandiphosphonsäure) oder [^{99m}Tc]HMDP (Hydroxymethandiphosphonsäure) werden in der Skelettszintigraphie von Knochenstoffwechselerkrankungen und Knochentumoren eingesetzt. Gegenüber der Skelettszintigraphie bzw. SPECT (Single Photon Computed Tomography) stellt die PET (Positronen Emissions Tomography) mit [¹⁸F]NaF jedoch eine deutlich sensitivere Methode zur Detektion von Knochentumoren dar. Radiomarkierte Bisphosphonate als PET-Tracer sind kommerziell nicht etabliert (E. Even-Sapir, U. Metser, G. Lievshitz, H. Lerman, I. Leibovitch, The Detection of Bone Metastases in Patients with High-Risk Prostate Cancer: 99mTc-MDP Planar Bone Scintigraphy, Single- and Multi-Field-of-View SPECT, 18F-Fluoride PET, and 18F-Fluoride PET/CT, J. Nucl. Med. 2006; 47: 287-297).

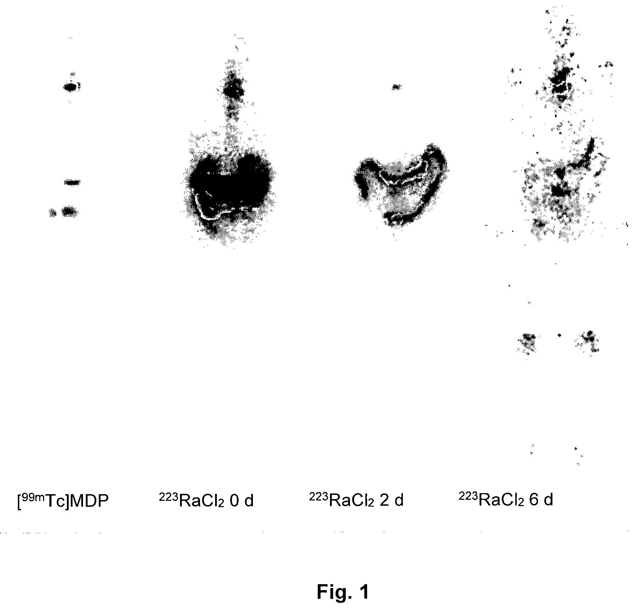

Einen weiteren Einsatzbereich von Radiometallen stellt die palliative Schmerztherapie von Knochentumoren dar. Hier werden insbesondere die Radioisotope von Metallen der zweiten Hauptgruppe wie ⁸⁹Sr(II) und ²²³Ra(II) eingesetzt. Diese Calciumanaloga werden mit hoher biologischer Halbwertszeit, ähnlich wie Calcium, bei der Mineralisierung des Knochens eingelagert. ²²³Ra(II) wurde 2013 als palliatives Endoradiotherapeutikum zur Behandlung von Knochentumoren zugelassen. Als problematisch zeigt sich bei diesen Calciummimetika allerdings die Ausscheidung der radioaktiven Substanz über den Darm (siehe Fig. 1: Vergleich von [^{99m}Tc]MDP und ²²³RaCl₂ an Tag 1, 2 und Tag 6 nach der Injektion: aus O. Sartor, P. Hoskin, Ø. S. Bruland, Targeted radio-nuclide therapy of skeletal metastases, Cancer Treatment Reviews, 2013; 39: 18-26).

Dreiwertige metallische Radionuklide wie ¹⁵³Sm(III), ¹⁷⁷Lu(III) und ⁹⁰Y(III) müssen mit Hilfe eines schwachen Chelators wie Citrat, EDTMP (Ethylenediamintetra[methylen phosphonsäure]) oder HEDTA ([Hydroxyethyl]-ethylen diamintriessigsäure) injiziert werden, um Komplexdissoziation und Leberakkumulation zu vermeiden. Stabilisierungen der dreiwertigen Radiolanthanide in Form makrozyklischer Chelatoren sind nicht etabliert. Dies ist insofern bemerkenswert, als z.B. das β-emittierende ¹⁷⁷Lu aufgrund seiner nuklearen Charakteristik (Halbwertzeit, Anteil und Energetik der β-Partikel), seiner kommerziellen Verfügbarkeit und der trägerfreien Konzentration auch kommerziell vielversprechend für therapeutische Ansätze für Knochentumore ist.

Im Bereich der quantitativen Bildgebung ist der Positronen-Emitter ⁶⁸Ga außerordentlich relevant, sowohl aus Gründen seiner Generator-basierten Verfügbarkeit, als auch aufgrund seiner nuklearen und chemischen Charakteristika. Als dreiwertiges Kation allein ist es nicht hinreichend selektiv bzgl. der Anreicherung an Knochentumoren. Typischerweise werden ⁶⁸Ga-Komplexe benötigt, bei denen die Liganden Phosphonat-Profile aufweisen, die eine Affinität zu HA und eine günstige Pharmakologie in den übrigen Organen vermitteln sollen.

Im Kontext von knochenaffinen Verbindungen wurde EDTMP mit ⁶⁸Ga(III) evaluiert, zeigte jedoch nur eine ungenügende Anreicherung im Zielgewebe und eignet sich folglich nur unzureichend als Diagnostikum (J. Goyal, E. S. Antonarakis, Bone-targeting radiopharmaceuticals for the treatment of prostate cancer with bone metastases, Cancer Letters, 2012; 323: 135-146; O. Sartor, P. Hoskin, Ø. S. Bruland, Targeted radio-nuclide therapy of skeletal metastases, Cancer Treatment Reviews, 2013; 39: 18-26; M. Mitterhauser, S. Toegela, W. Wadsak, R. Klugerg, H. Viernstein , R. Dudczaka, K. Kletter, Pre vivo, ex vivo and in vivo evaluations of [68Ga]EDTMP, Nuclear Medicine and Biology, 2007; 34: 391-397).

Im aktuellen Fokus der Forschung befinden sich makrozyklische Liganden, vor allem DOTA (1,4,7,10-Tetraazacyclododecan-1,4,7,10-tetraessigsäure), DOTAM (1,4,7,10-Tetrakis(carbamoylmethyl)-1,4,7,10-tetraazacyclododecan) bzw. NOTA (1,4,7-triazacyclononan-1,4,7-triessigsäure) als konjugierte Bisphosphonate. Diese eignen sich auf Grund ihres makrozyklischen bifunktionellen Chelators zur stabilen Komplexierung unterschiedlichster Radiometalle, wie beispielsweise ⁶⁸Ga(III) und dem Therapienuklid ¹⁷⁷Lu(III). Unter den in der Fachliteratur wie auch Patentschriften beschriebenen DOTA-Bisphosphonaten, bzw. NOTA-Bisphosphonaten befinden sich ausschließlich α-H-Bisphosphonate und α-Hydroxy-Bisphosphonate, jedoch keine N-heteroaromatischen Verbindungen (M. Meckel, M. Fellner, N. Thieme, R. Bergmann, V. Kubicek, F. Rösch, In vivo comparison of DOTA based 68Ga-labelled bisphosphonates for bone imaging in non-tumour models, Nucl. Med. Biol., 2013; 40: 823-830; K. Ogawa, K.Takai, H. Kanbara, T. Kiwada, Y. Kitamura, K. Shiba, A. Odani, Preparation and evaluation of a radiogallium complex-conjugated bisphosphonate as a bone scintigraphy agent, Nuc. Med. Biol., 2011; 38: 631-636; US 2012/0148492 A1, Jun. 14, 2012, Bisphosphonic acid derivative and compound thereof labeled with radioactive metal nuclide, Hiroyuki Dozono, Fujifilm RI Pharma Co. Ltd., Tokyo, Japan).

Die im Stand der Technik bekannten N-haltigen α-H-Bisphosphonate bzw. α-Hydroxy-Bisphosphonate verwenden DOTA oder NOTA als Chelator, wobei die geminalen Bisphosphonate über eine Amidbindung an das Stickstoffatom in C₃₋₄-Position des Chelators konjugiert sind. Die bekannten Konjugate N-haltiger α-H-Bisphosphonate bzw. α-Hydroxy-Bisphosphonate haben allerdings eine im Vergleich zum geminalen Bisphosphonat stark verringerte Affinität zu Knochensubstanz. Es wird vermutet, dass die Derivatisierung des jeweiligen geminalen Bisphosphonats dessen Affinität erheblich mindert. Andere N-heteroaromatische α-Hydroxy-Bisphosphonate, wie beispielsweise Zoledronat, welches eine hohe Affinität zur Knochensubstanz aufweisen, konnten bisher nicht mit einem bifunktionellen Chelator konjugiert werden.

Die Erfindung hat die Aufgabe, ein Pharmakon mit einer im Vergleich zum Stand der Technik erhöhten Akkumulation an Knochen, insbesondere an Knochentumoren, bereitzustellen. Hierbei soll ein besseres Anreicherungsverhältnis für Knochen-zu-Blut und Knochen-zu-Weichteilgewebe, eine höhere Bindungsausbeute zu Knochentumoren sowie eine effiziente renale Ausscheidung von nichtgebundenem Pharmakon erzielt werden. Je nach Wahl des Radionuklids sollen die erfindungsgemäßen Bisphosphonat-Derivate für die molekulare Bildgebung, insbesondere als PET-Diagnostikum, und als Endoradiotherapeutikum einsetzbar sein.

Diese Aufgabe wird gelöst durch eine Verbindung V, umfassend einen Chelator X und einen oder mehrere, mit dem Chelator X konjugierte Targetingvektoren (TV) mit der Struktur -L₁-R₁-L₂-R₂-L₃-R₃, wobei
L₁ aus der Gruppe, umfassend Amid, Phosphinat, Alkyl, Triazol, Thioharnstoff, Ethylen, Maleimid, -(CH₂)ₖ- und -(CH₂CH₂O)ₖ- mit k = 1, 2, 3, 4, 5, 6, 7, 8, 9 oder 10 gewählt ist,
L₂ aus -(CH₂)ₘ- und -(CH₂CH₂O)ₘ- mit m = 1, 2, 3, 4, 5, 6, 7, 8, 9 oder 10 gewählt ist, und
L₃ aus -(CH₂)ₙ- und -(CH₂CH₂O)ₙ- mit n = 1, 2, 3, 4, 5, 6, 7, 8, 9 oder 10 gewählt ist, und 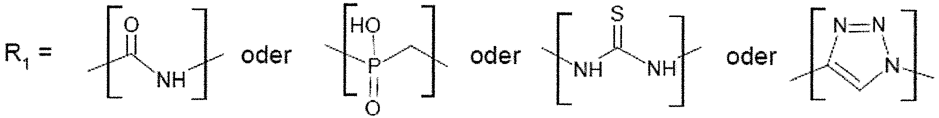
R2 gewählt ist aus der Gruppe, umfassend eine Restgruppe eines: Pyrrols, Pyridins, Pyrimidins, Furans, Azols, Triazols, Tetrazols, Pyrazols, Imidazols, Oxazols, Oxadiazols, Thiophens, Thiazols, Thiadiazols, Azins, Oxazins, Thiazins, Naphthalins, Chinolins, Chromens oder Thiochromens;
   und 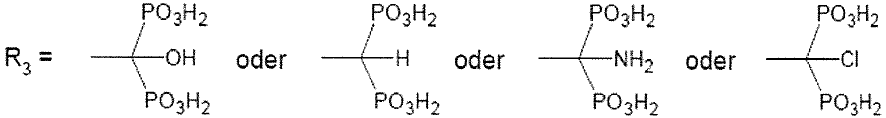
ist.

Der Begriff "Chelator" bzw. die Bezeichnung "X" bezeichnet hierin allgemein eine Verbindung, die befähigt ist, ein Metallion zu komplexieren. Bevorzugte Chelatoren sind zyklische Verbindungen, die optional mit einer oder mehreren Seitenketten versehen sind. Solche zyklischen Verbindungen mit oder ohne Seitenkette(n) werden auch als "makrozyklische Liganden" oder "makrozyklische Chelatoren" bezeichnet. Ein beispielhafter makrozyklischer Chelator mit vier Seitenketten ist DOTA: Er besteht aus dem 12-gliedrigen Makrozyklus 1,4,7,10-Tetraazacyclododecan, der an den vier Stickstoffatomen des Makrozyklus, also den Positionen 1,4,7 und 10 des Makrozyklus, durch Essigsäure-Reste substituiert ist: also 1,4,7,10-Tetraazacyclododecan-1,4,7,10-tetraessigsäure.

Der Begriff "Derivat", bezogen auf einen Chelator X, bezeichnet eine Verbindung, die sich von diesem Chelator X dadurch unterscheidet, dass mindestens eine Gruppe des Chelators X durch eine andere Gruppe ersetzt ist. In Bezug auf ein Derivat eines Chelators kann synonypm ausgedrückt werden, dass das Derivat von dem Chelator "abgeleitet" ist. Solche Verbindung werden in Bezug auf den Chelator X hierin als dessen Derivate bzw. deren Derivate bezeichnet. Handelt es sich bei dem Chelator X um einen makrozyklischen Liganden, so unterscheidet sich das Derivat vom Chelator entweder (a) dadurch, dass mindestens eine Gruppe, die Bestandteil des Makrozyklus ist, durch eine andere Gruppe ersetzt ist (z.B. kann eine Methylen- durch eine Ethylengruppe ersetzt sein oder umgekehrt), und/oder (b) dadurch, dass mindestens eine Gruppe, die eine Seitenkette oder ein Bestandteil einer Seitenkette ist, durch eine andere Gruppe ersetzt ist (z.B. kann eine Essigsäure- durch eine Essigsäureamidgruppe ersetzt sein oder umgekehrt). Derivate mit einem Unterschied vom Typ (b) sind bevorzugt. Darunter weiter bevorzugt sind Derivate, mit einem Unterschied vom Typ (b), aber ohne einen Unterschied vom Typ (a). Ein beispielhaftes Derivat des Chelators DOTA ist die Verbindung DOTAM, bei der alle vier Essigsäuregruppen-Seitenketten von DOTA durch Essigsäureamidgruppen ersetzt sind. Das Derivat ist bevorzugt selbst ein Chelator, d.h. eine Verbindung, die befähigt ist, ein Metallion zu komplexieren.

Zweckmäßige Ausführungsformen der Erfindung sind dadurch gekennzeichnet, dass:
- der Chelator X gewählt ist aus der Gruppe, umfassend EDTA (Ethylendiamintetraacetat), EDTMP (Diethylentriaminpenta(methylenphosphonsäure)), DTPA (Diethylentriaminpentaessigsäure) und dessen Derivate, DOTA (1,4,7,10-Tetraazacyclododecan-1,4,7,10-tetraessigsäure), DOTAGA (Dodeca-1-glutarsäure-1,4,7,10-tetraamin-triessigsäure), DOTAM (1,4,7,10-Tetrakis(carbamoylmethyl)-1,4,7,10-tetraazacyclododecan) und anderen DOTA-Derivaten, TRITA (Trideca-1,4,7,10-tetraamin-tetraessigsäure), TETA (Tetradeca-1,4,8,11-tetraamin-tetraessigsäure) und dessen Derivate, NOTA (Nona-1,4,7-triamin-triessigsäure) und dessen Derivate wie beispielsweise NOTAGA (1,4,7-triazacyclononan,1-glutarsäure,4,7-essigsäure), NOPO (1,4,7-triazacyclononan-1,4-bis[methylen(hydroxymethyl)phosphinsäure]-7-[methylen(2-carboxyethyl)phosphinsäure]), PEPA (Pentadeca-1,4,7,10,13-pentaaminpenta-essigsäure), HEHA (Hexadeca-1,4,7,10,13,16-hexaamin-hexaessigsäure) und dessen Derivate, HBED (Hydroxybenzyl-ethylen-diamin) und dessen Derivate, DEDPA und dessen Derivate, wie H₂DEDPA (1,2-[{6-(carboxylat-)pyridin-2-yl}methylamin]ethan), DFO (Deferoxamin) und dessen Derivate, Deferipron, CP256 (4-Acetylamino-4-{2-[(3-hydroxy-1,6-dimethyl-4-oxo-1,4-dihydro-pyridin-2-ylmethyl)-carbamoyl]-ethyl}-heptandisäure bis-[(3-hydroxy-1,6-dimethyl-4-oxo-1,4-dihydro-pyridin-2-ylmethyl)-amid]) und dessen Derivate, wie YM103; TRAP (Triazacyclononan-phosphinsäure), TEAP (Tetraazycyclodecan-phosphinsäure) und dessen Derivate, AAZTA (6-Amino-6-methylperhydro-1,4-diazepin-N,N,N',N'-tetraessigsäure) und Derivate wie DATA; SarAr (1-N-(4-aminobenzyl)-3,6,10,13,16,19-hexaazabicyclo[6.6.6]-eicosan-1,8-diamin) und Salze davon;
- die Verbindung V, die eine Struktur gemäß der Formel I aufweist 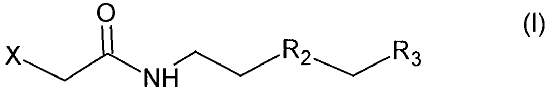 worin X den Chelator bezeichnet; und/oder
- die Verbindung V, die eine Struktur gemäß der Formel II aufweist, 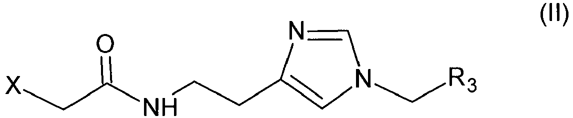
worin X den Chelator bezeichnet.

Die Erfindung hat zudem die Aufgabe, ein Pharmakon für die Behandlung von Knochenerkrankungen bereitzustellen. Diese Aufgabe wird gelöst durch ein Pharmakon, das die vorstehend beschriebene Verbindung V und ein mit der Verbindung V komplexiertes metallisches Isotop M umfasst. Vorzugsweise ist das metallische Isotop M gewählt aus der Gruppe, umfassend ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ^{99m}Tc, ¹¹¹In, ¹³⁵Sm, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi und ²²⁵Ac. Besonders bevorzugt sind ⁶⁶Ga, ⁶⁷Ga, ⁶⁸Ga und ¹⁷⁷Lu.

In dieser Erfindung sind besonders bevorzugt diejenigen Kombinationen aus einem bevorzugten metallischen Isotop und/oder einem bevorzugten Chelator X und/oder einer bevorzugte Verbindung V, mit einem bevorzugten Bisphosphonat.

Eine weitere Aufgabe der vorliegenden Erfindung besteht darin, ein Verfahren zur Herstellung eines Pharmakons aus der Verbindung V und einem metallischen Isotop M bereit zu stellen.

Diese Aufgabe wird gelöst durch ein Verfahren, welches die folgenden Schritte umfasst:
(a) Bereitstellen einer die Verbindung V nach einem der Ansprüche 1 bis 7 enthaltenden Lösung S;
(b) Bereitstellen eines metallischen Isotops M, wie beispielsweise ⁶⁸Ga(III); und
(c) Ligieren des metallischen Isotops M mit der Verbindung V unter Bildung eines Komplexes MV des metallischen Isotops M mit der Verbindung V in einer Lösung F.

Zweckmäßige Ausführungsformen des erfindungsgemäßen Verfahrens sind dadurch gekennzeichnet, dass:
- in Schritt (b) das metallische Isotop M in einer Lösung bereitgestellt wird;
- in Schritt (b) ein Radionuklidgenerator mit einem Mutternuklid und einem durch Zerfall des Mutternuklids erzeugten metallischen Isotop M bereitgestellt und in Schritt (c) das metallische Isotop M mit der Lösung S von dem Mutternuklid separiert wird;
- in Schritt (b) das metallische Isotop M in einem Ionenaustauscher enthalten ist und in Schritt (c) das metallische Isotop M mit der Lösung S von dem Ionenaustauscher eluiert wird;
- in Schritt (b) das metallische Isotop M in einem Ionenaustauscher enthalten ist und in Schritt (c) das metallische Isotop M mit einem Lösungsmittel E von dem Ionenaustauscher eluiert wird, um eine das metallische Isotop M enthaltende Lösung ME zu erhalten, und die Lösung ME mit der Lösung S gemischt wird, um die Lösung F mit dem Komplex MV zu erhalten;
- in Schritt (c) vor der Elution des metallischen Isotops M der Ionenaustauscher mit einem oder mehreren Lösungsmitteln gespült wird, um Verunreinigungen zu entfernen;
- der Ionenaustauscher ein Kationenaustauscher ist;
- der Ionenaustauscher als wirksame Komponente sulfoniertes Poly(styrol-co-divinylbenzol)-Harz umfasst, wobei das Poly(styrol-co-divinylbenzol)-Harz Divinylbenzol in einer Menge von 2 bis 20 mol-% basierend auf 100 mol-% Styrol- und Divinylbenzol-Monomereinheiten enthält;
- die Lösung F im Anschluss an Schritt (c) filtriert und/oder neutralisiert wird;
- Schritt (c) innerhalb von 6 s bis 5 min, innerhalb von 6 s bis 3 min, innerhalb von 6 s bis 2 min oder vorzugsweise innerhalb von 6 s bis 1 min, abgeschlossen ist;
- Schritt (c) bei einer Temperatur von 10 bis 95 °C, von 10 bis 90 °C, von 10 bis 40 °C oder vorzugsweise von 10 bis 30 °C durchgeführt wird;
- in Schritt (b) ein Radionuklidgenerator eingesetzt wird, wobei der Radionuklidgenerator ein auf einer chromatographischen Säule adsorbiertes Mutternuklid, wie beispielsweise ⁶⁸Ge, umfasst und ein durch Zerfall des Mutternuklids gebildetes Tochternuklid, wie beispielsweise ⁶⁸Ga, von der chromatographischen Säule eluiert wird;
   und/oder
- in Schritt (b) ein Radionuklidgenerator eingesetzt wird, wobei der Radionuklidgenerator eine Lösung mit einem Mutternuklid, wie beispielsweise ⁹⁰Sr enthält und ein durch Zerfall des Mutternuklids erzeugtes Tochternuklid, wie beispielsweise ⁹⁰Y, aus der Lösung eluiert wird.

Die erfindungsgemäße Verbindung V umfasst einen Chelator X und einen oder mehrere, mit dem Chelator X konjugierte Targetingvektoren mit der Struktur -L₁-R₁-L₂-R₂-L₃-R₃. Insbesondere weist die Verbindung V eine der folgenden Strukturen auf: 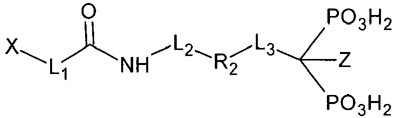 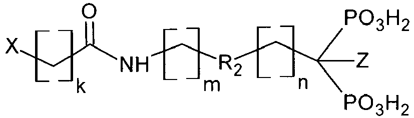 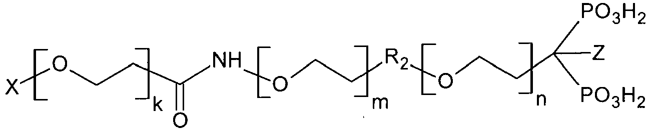 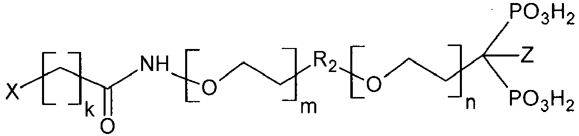 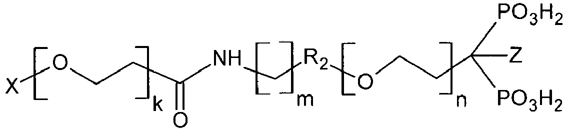 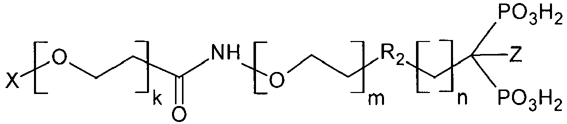 mit
k = 1, 2, 3, 4, 5, 6, 7, 8, 9 oder 10;
m = 1, 2, 3, 4, 5, 6, 7, 8, 9 oder 10;
n = 1, 2, 3, 4, 5, 6, 7, 8, 9 oder 10; und
Z = OH, H, NH₂ oder Cl.

R₂ ist vorzugsweise ausgewählt aus der Gruppe, umfassend die Restgruppen der Moleküle: 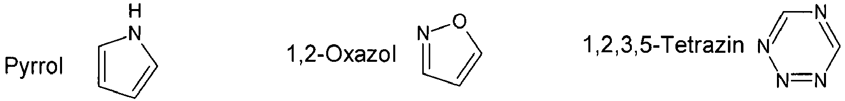 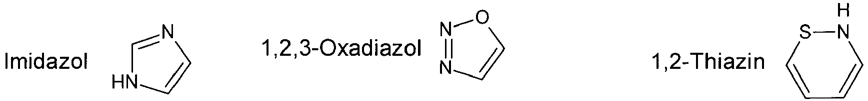 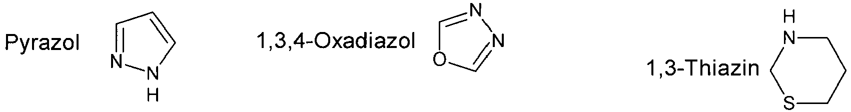 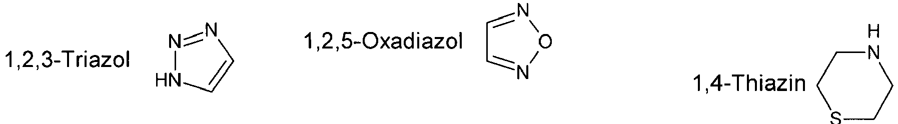 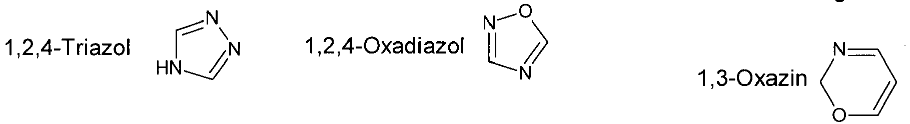 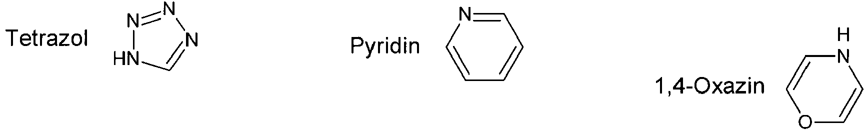 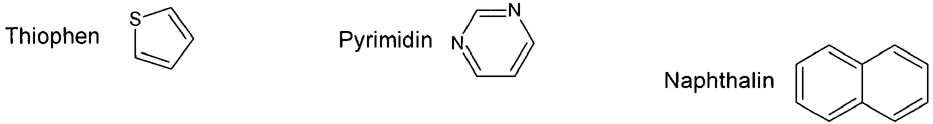 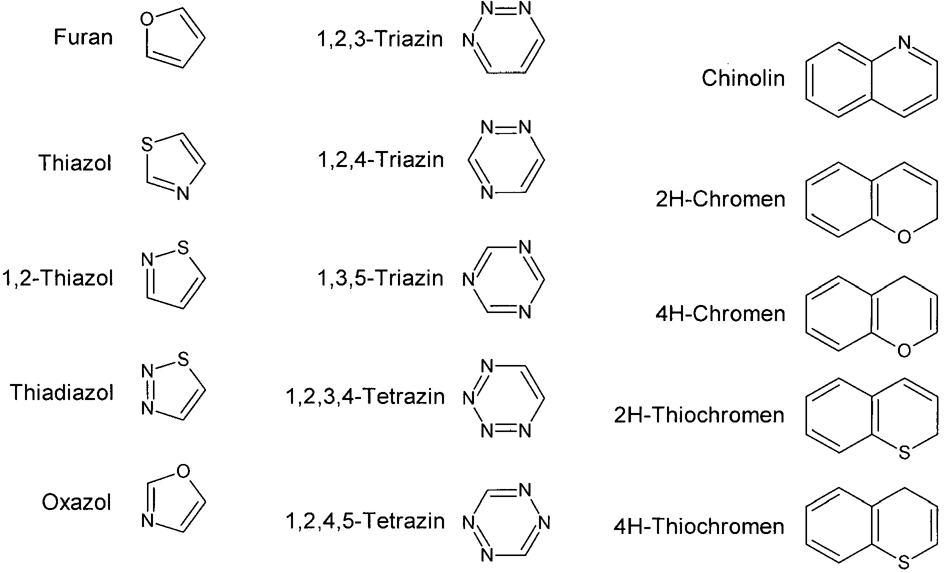 und Isomere der vorstehenden Moleküle.

Dementsprechend hat die erfindungsgemäße Verbindung V eine zu den nachfolgend gezeigten Verbindungen identische oder analoge Struktur, wobei das Molekül, von dem sich die Gruppe R₂ ableitet, jeweils angegeben ist:

| **Molekül, von dem R₂ abgeleitet ist** | **Verbindung V** | **Struktur Nr.** |
|---|---|---|
| Pyrrol | 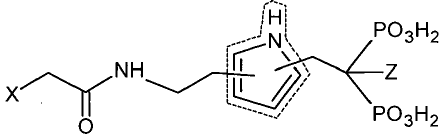 | (IV) |
| Imidazol | 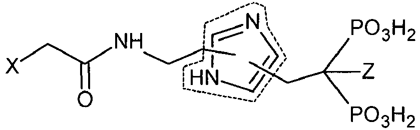 | (V) |
| Pyrazol | 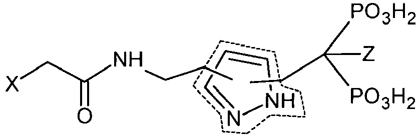 | (VI) |
| 1,2,3-Triazol | 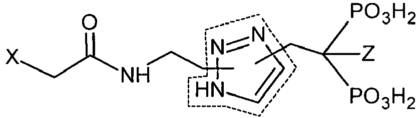 | (VII) |
| 1,2,4-Triazol | 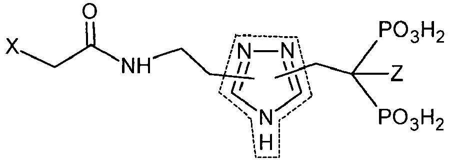 | (VIII) |
| Tetrazol | 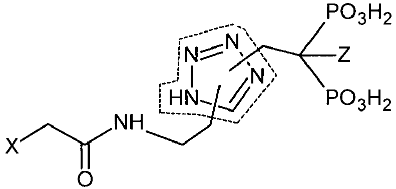 | (IX) |
| Thiophen | 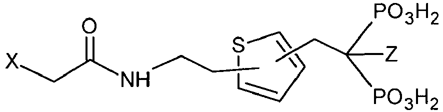 | (X) |
| Furan | 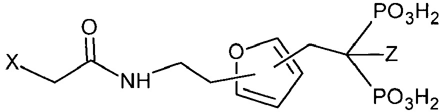 | (XI) |
| Thiazol | 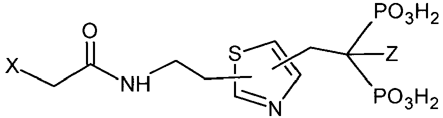 | (XII) |
| 1,2-Thiazol | 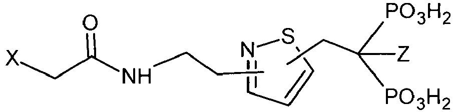 | (XIII) |
| Thiadiazol | 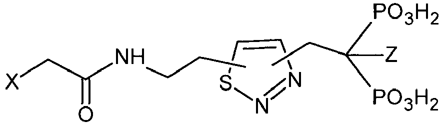 | (XIV) |
| Oxazol | 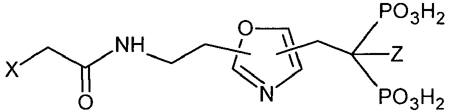 | (XV) |
| 1,2-Oxazol | 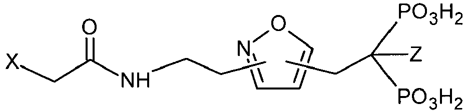 | (XVI) |
| 1,2,3-Oxadiazol | 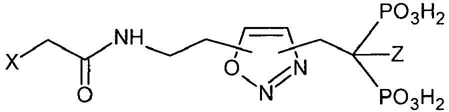 | (XVII) |
| 1,3,4-Oxadiazol | 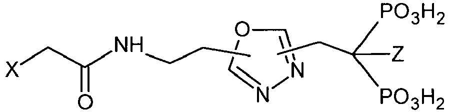 | (XVIII) |
| 1,2,5-Oxadiazol | 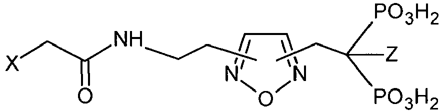 | (XIX) |
| 1,2,4-Oxadiazol | 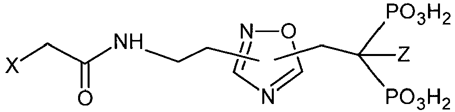 | (XX) |
| Pyridin | 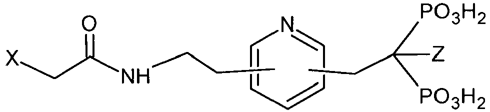 | (XXI) |
| Pyrimidin | 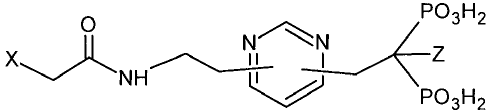 | (XXII) |
| 1,2,3-Triazin | 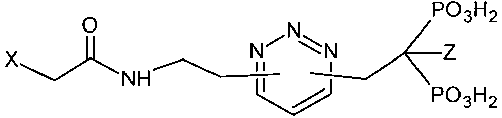 | (XXIII) |
| 1,2,4-Triazin | 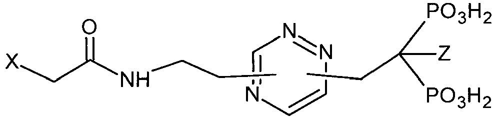 | (XXIV) |
| 1,3,5-Triazin | 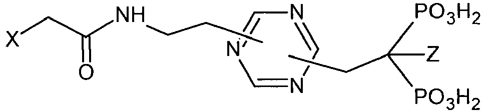 | (XXV) |
| 1,2,3,4-Tetrazin | 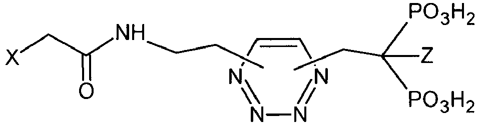 | (XXVI) |
| 1,2,4,5-Tetrazin | 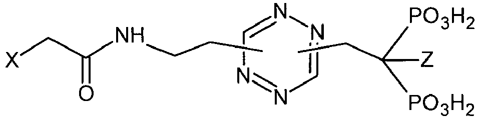 | (XXVII) |
| 1,2,3,5-Tetrazin | 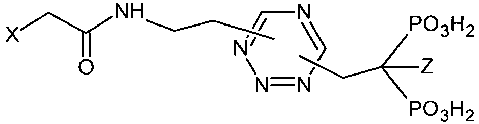 | (XXVIII) |
| 1,2-Thiazin | 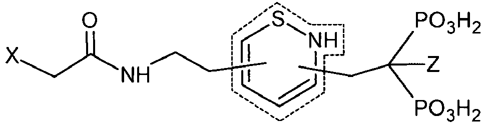 | (XXIX) |
| 1,3-Thiazin | 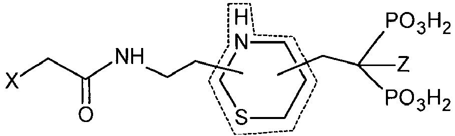 | (XXX) |
| 1,4-Thiazin | 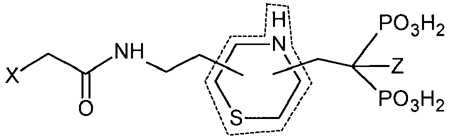 | (XXXI) |
| 1,3-Oxazin | 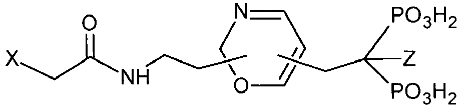 | (XXXII) |
| 1,4-Oxazin | 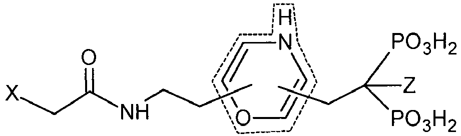 | (XXXIII) |
| Napthalin | 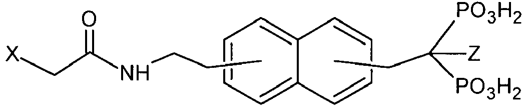 | (XXXIV) |
| Chinolin | 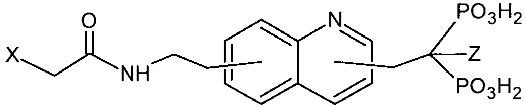 | (XXXV) |
| 2H-Chromen | 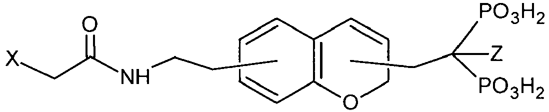 | (XXXVI) |
| 4H-Chromen | 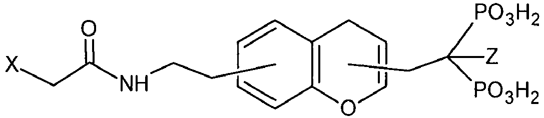 | (XXXVII) |
| 2H-Thiochromen | 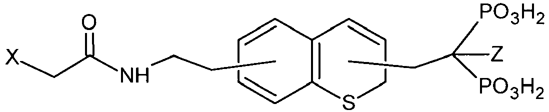 | (XXXVIII) |
| 4H-Thiochromen | 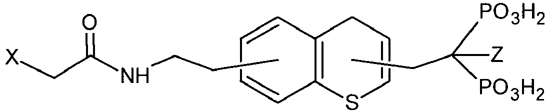 | (XXXIX) |

In einigen der vorstehend gezeigten erfindungsgemäßen Verbindungen beziehungsweise Strukturformeln ist die Gruppe R₂ mit einer gestrichelten Linie umrandet, die eine Markush-Schattierung repräsentiert und anzeigt, dass jede der möglichen Bindungstellen der Gruppe R₂ für die Bindung zwischen R₂ und der Gruppe R₃ bzw. -L₃-R₃ (d. h. dem Bisphosphonat) sowie für die Bindung zwischen R₂ und der Gruppe R₁ bzw. -R₁-L₂- genutzt wird. Insbesondere können die Gruppen -R₁-L₂- und/oder -L₃-R₃ auch über ein Stickstoffatom der NH-Substituenten der Gruppe R₂ gebunden sein, wobei die Bindung das Wasserstoffatom des NH-Substituenten ersetzt.

In den vorstehenden Strukturformeln ist:
L₁ = -(CH₂)ₖ- mit k = 1;
L2 = -(CH₂)ₘ- mit m = 2;
L₃ = -(CH₂)ₙ- mit n = 1; und
Z = OH, H, NH₂ oder Cl.

Im Rahmen der Erfindung sind zudem Verbindungen vorgesehen, in denen

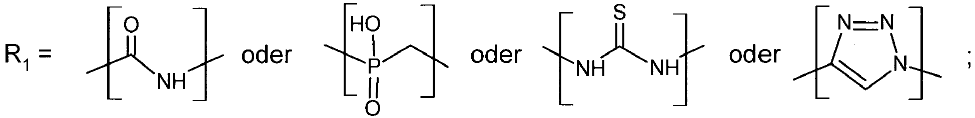

L₁ gewählt ist aus der Gruppe, umfassend einen Amid-, Phosphinat-, Alkyl-, Triazol-, Thioharnstoff-, Ethylen-, Maleimid-Rest und -(CH₂)ₖ- oder -(CH₂CH₂O)ₖ- mit k = 1, 2, 3, 4, 5, 6, 7, 8, 9 oder 10;
L₂ = -(CH₂)ₘ- oder -(CH₂CH₂O)ₘ- mit m = 1, 2, 3, 4, 5, 6, 7, 8, 9 oder 10; undr
L₃ = -(CH₂)ₙ- oder -(CH₂CH₂O)ₙ- mit n = 1, 2, 3, 4, 5, 6, 7, 8, 9 oder 10.

Nachfolgend ist die Basisstruktur einiger der erfindungsgemäßen Chelatoren X gezeigt.

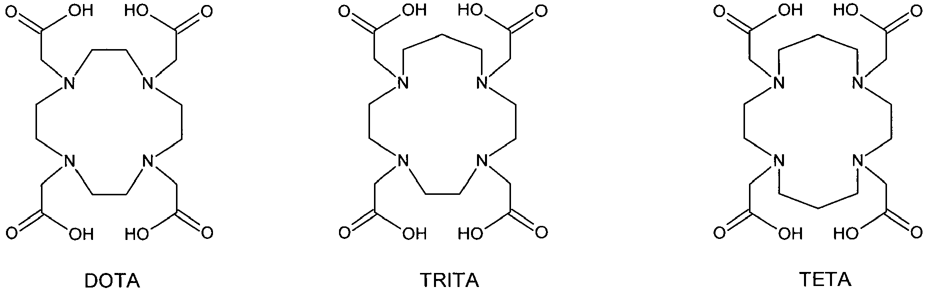

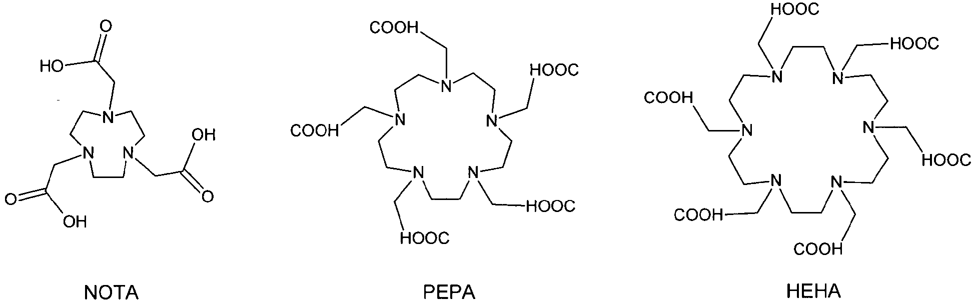

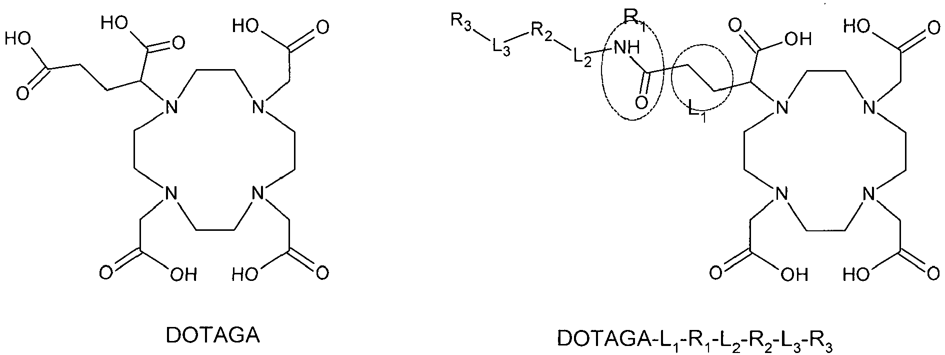

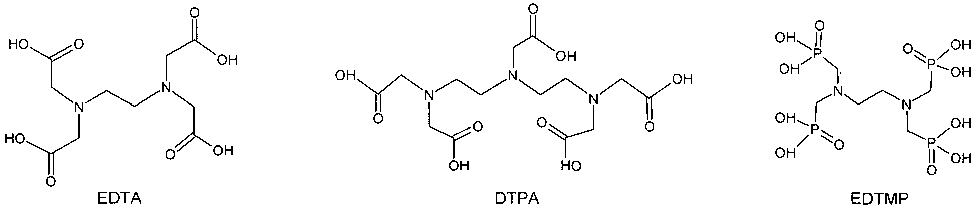

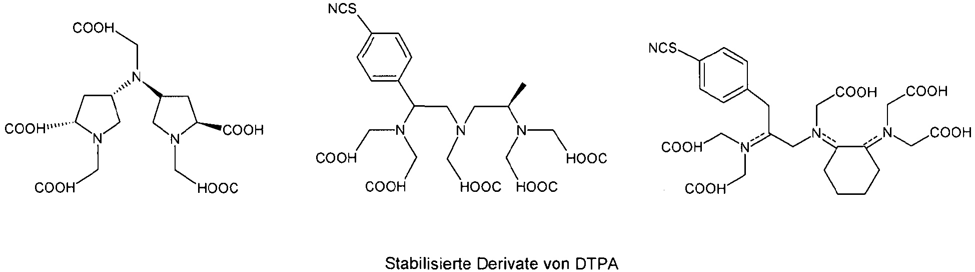

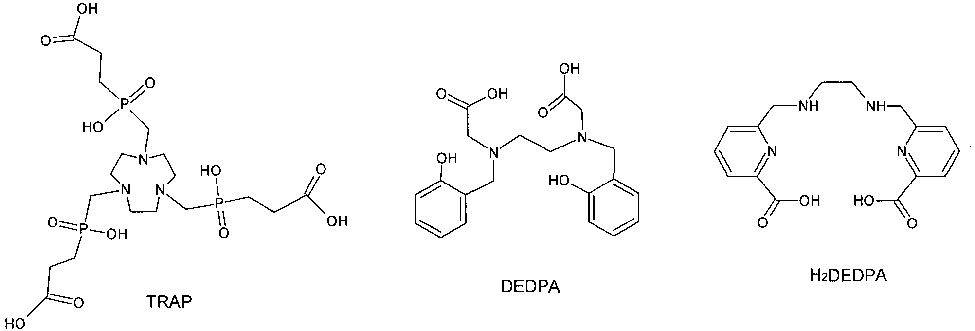

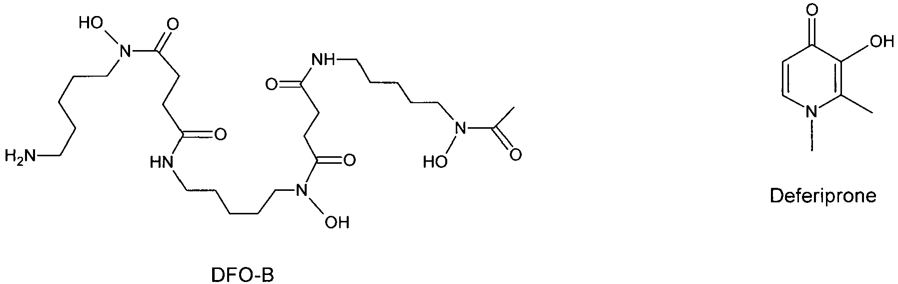

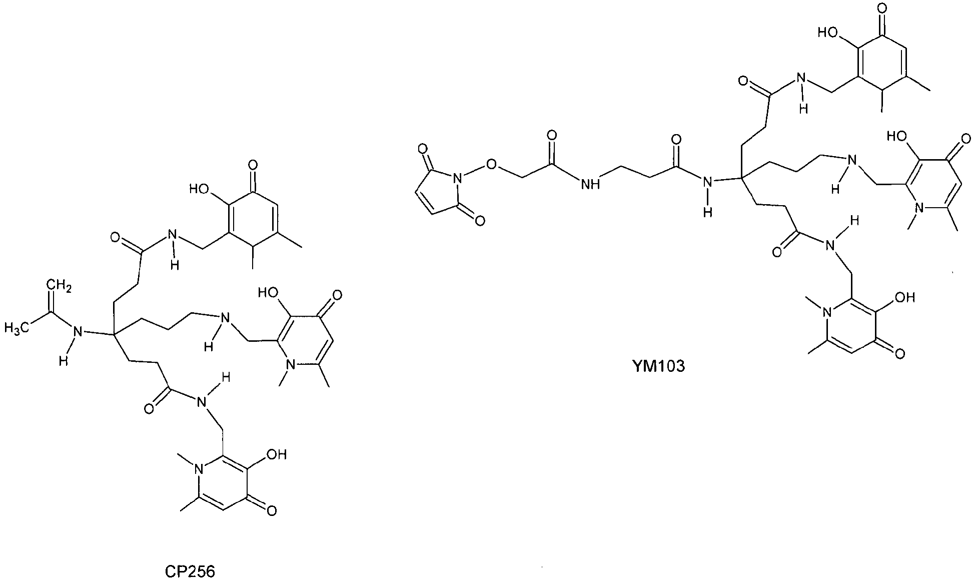

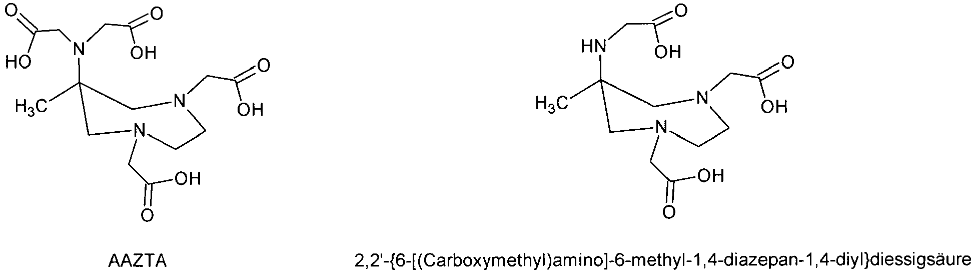

Eine erfindungsgemäß besonders bevorzugte Verbindung V ist DOTA^{ZOL} mit der Strukturformel III

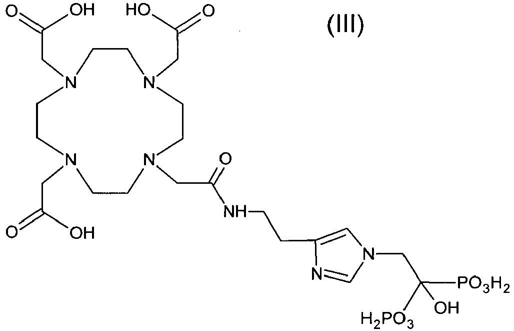

Eine erfindungsgemäß ebenso besonders bevorzugte Verbindung V ist DOTAM^{ZOL}.

Auf DOTA^{ZOL} basierende Pharmaka des Typs [M]DOTA^{ZOL}, wie beispielsweise [⁶⁸Ga]DOTA^{ZOL} und [¹⁷⁷Lu]DOTA^{ZOL} sowie alle davon abgeleiteten Derivate (z.B. DOTAM-basierte Derivate) lassen sich effizient herstellen und sind chemisch und unter physiologischen Bedingungen stabil. In den vorstehenden Strukturformeln ist die Komplexierung des metallischen Isotops M durch eckige Klammern symbolisiert.

Diese Erfindung bezieht sich auch auf Vorrichtungen zum Herstellen eines Radiopharmakons.

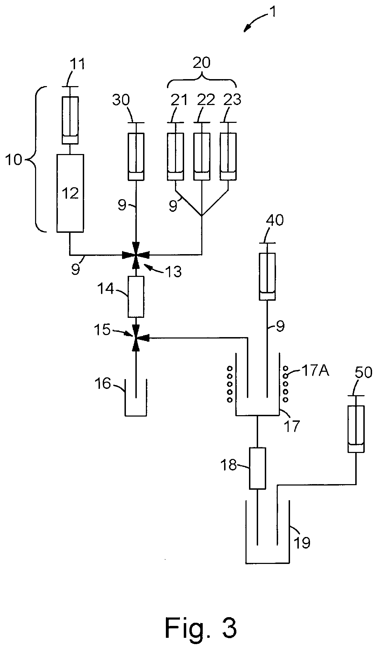

Fig. 3 zeigt schematisch eine erste Vorrichtung 1 zur Herstellung eines Radiopharmakons aus der erfindungsgemäßen Verbindung V und einem metallischen Radioisotop M, welches vorzugsweise gewählt ist aus ⁶⁶Ga, ⁶⁷Ga und ⁶⁸Ga. Vorrichtung 1 umfasst einen Radionuklidgenerator 10 mit einer chromatographischen Säule 12, an der ein metallisches Mutternuklid, wie ⁶⁸Ge adsorbiert ist, und einer Elutionsvorrichtung 11 zum Eluieren eines durch Zerfall des Mutternuklids erzeugten Tochternuklids M, wie ⁶⁸Ga, von der chromatographischen Säule 12 mit einem geeigneten Lösungsmittel. Die Elutionsvorrichtung 11 umfasst beispielsweise einen Vorlagebehälter für das Lösungsmittel und eine Pumpe. Die verschiedenen Komponenten von Vorrichtung 1 sind über Fluidleitungen, die in Fig. 3 durchgängig mit dem Bezugszeichen 9 bezeichnet sind, miteinander verbunden. Der Auslass der chromatographischen Säule 12 ist über eine Fluidleitung und ein erstes Mehrwegeventil 13 mit dem Einlass eines Ionenaustauschers 14 verbunden. Der Ionenaustauscher 14 umfasst vorzugsweise sulfoniertes Poly(styrol-co-divinylbenzol)harz als aktive Komponente, wobei das Poly(styrol-co-divinylbenzol)harz Divinylbenzol in einer Menge von 2 bis 20 mol-%, basierend auf 100 mol-% Styrol- und Divinylbenzolmonomereinheiten, umfasst. Das von der chromatographischen Säule 12 eluierte Tochternuklid M wird auf dem Ionenaustauscher 14 adsorbiert, während das ebenfalls eluierte Mutternuklid nicht adsorbiert und praktisch vollständig über ein zweites Mehrwegeventil 15 in ein Auffanggefäß 16 geführt wird.

Die Vorrichtung 1 umfasst vorzugsweise weitere Elutionsvorrichtungen 21, 22, 23, die über Fluidleitungen und das erste Mehrwegeventil 13 mit dem Einlass des Ionenaustauschers 14 verbunden sind. Die Elutionsvorrichtungen 21, 22, 23 dienen zum Reinigen des Ionenaustauschers 14 bzw. des an dem Ionenaustauscher 14 adsorbierten Tochternuklids M. Insbesondere werden mittels der Elutionsvorrichtungen 21, 22, 23 Rückstände, die aus dem Radionuklidgenerator 10 stammen, wie das Mutternuklid, Fe^{III}, Zn^{II} und Ti^{IV} entfernt. Die einen oder mehreren Eluate bzw. Lösungsmittel, die den Ionenaustauscher 14 verlassen, werden ebenfalls über das zweite Mehrwegeventil 15 in das Auffanggefäß 16 geleitet.

Eine weitere Elutionsvorrichtung 30, die über eine Fluidleitung und das erste Mehrwegeventil 13 mit dem Einlass des Ionenaustauschers 14 verbunden ist, wird zum Eluieren des gereinigten Tochternuklids M von dem Ionenaustauscher 14 eingesetzt. Das Eluat mit dem Tochternuklid M wird über das zweite Mehrwegeventil 15 in ein Reaktionsgefäß 17 geleitet. Das Reaktionsgefäß 17 ist vorzugsweise mit einer elektrischen Heizvorrichtung 17A ausgestattet. Aus einem Vorlagebehälter 40 wird eine die erfindungsgemäße Verbindung V enthaltende Lösung in das Reaktionsgefäß 17 geleitet, wodurch die Komplexierung des Tochternuklids M mit der Verbindung V eingeleitet wird. Nach Abschluss der Komplexierung wird die radioaktiv markierte Verbindung VM, d.h. das aus der Verbindung V und dem ligierten Tochternuklid M bestehende Radiopharmakon, über einen optionalen Filter 18 in ein Produktgefäß 19 geleitet, wo es gegebenenfalls mit einer aus einem Vorlagebehälter 50 zugeführten Lösung neutralisiert wird.

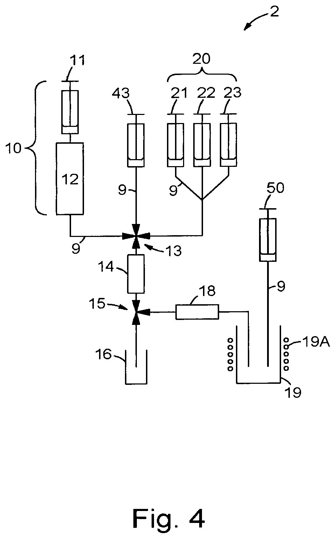

Fig. 4 zeigt eine zweite Vorrichtung 2 zur Herstellung eines Radiopharmakons aus der erfindungsgemäßen Verbindung V und einem metallischen Radioisotop M, das vorzugsweise gewählt ist aus ⁶⁶Ga, ⁶⁷Ga und ⁶⁸Ga. In Fig. 4 bezeichnen mit Fig. 3 übereinstimmende Bezugszeichen Komponenten mit der gleichen Funktion. Die zweite Vorrichtung 2 unterscheidet sich von der Vorrichtung 1 darin, dass eine die erfindungsgemäße Verbindung V enthaltende Lösung aus einer Elutionsvorrichtung 43 über eine Fluidleitung und ein Mehrwegeventil 13 mit dem Einlass eines Ionenaustauschers 14 verbunden ist. Die Lösung mit der Verbindung V wird dem Ionenaustauscher 14 direkt zugeführt, wobei das Tochternuklid M zugleich eluiert und komplexiert wird. Von dem Ionenaustauscher 14 wird das Eluat mit der erfindungsgemäßen Verbindung V und dem metallischen Radioisotop M über das Mehrwegeventil 15 und einen optionalen Filter 18 in ein Produktgefäß 19 geleitet. In Fällen, in denen die erfindungsgemäße Verbindung V das Tochternuklid M effizient und stabil komplexiert, kann das Radiopharmakon mittels der Vorrichtung 2 schnell und effektiv hergestellt werden.

Fig. 3 und 4 zeigen spezielle Vorrichtungen mit einem Radionuklidgenerator und einem Ionenaustauscher. Ein separater Ionenaustauscher ist jedoch nicht immer erforderlich. Vielmehr ist es für zahlreiche Anwendungen zweckmäßig, den Ionenaustauscher als Adsorber in dem Radionuklidgenerator einzusetzen.

In weiteren Anwendungsfällen bzw. Ausführungsformen ist weder ein Radionuklidgenerator noch ein Ionenaustauscher erforderlich. In diesen Anwendungsfällen bzw. Ausführungsformen wird das metallische Isotop bzw. metallische Radioisotop in einer Lösung bereitgestellt.

### Medizinische Verwendungen und andere damit in Beziehung stehende Verwendungen

Die vorliegende Erfindung bezieht sich auch auf die Verwendung einer erfindungsgemäßen Verbindung V als Markierungsvorläufer zur Herstellung eines Medikaments bzw. Pharmakons. Die Erfindung bezieht sich auch auf die Verwendung eines solchen Pharmakons in einem bildgebenden Verfahren mittels Positronen-Emissions-Tomographie oder Einzelphotonen-Emissionscomputertomographie. Das erfindungsgemäße Pharmakon ist geeignet zur Verwendung in einem Behandlungs- oder Therapieverfahren. Insbesondere ist es erfindungsgemäß geeignet zur Verwendung in einem Verfahren zur Behandlung von Knochenerkrankungen und Knochentumoren. Solche Verfahren umfassen insbesondere Verfahren zur Verwendung des Pharmakons bei der Behandlung von Erkrankungen an nicht-manifestierten Knochenmetastasen. Diese Verfahren umfassen bevorzugt die Anreicherung in der Tumorzelle, um die Farnesyl-Pyrophosphat-Synthase (FPPS) zu hemmen. Andere medizinische Verfahren und Verwendungen des Pharmakons gemäß der Erfindung umfassen die Abbildung von pharmakokinetischen Vorgängen, wie Herzerkrankungen, mittels Positronen-Emissions-Tomographie oder Einzelphotonen-Emissionscomputertomographie. Das Pharmakon kann auch *in vivo* oder ex *vivo* verwendet werden als Additiv in künstlicher Knochensubstanz, in Knochenzement oder in Knochenimplantaten.

Die Erfindung bezieht sich weiterhin auf die Verwendung einer erfindungsgemäßen Verbindung V in Verbindung mit einem metallischen Isotop M, wie Gadolinium, zur Herstellung eines Pharmakons, wobei das metallische Isotop M bevorzugt unter den Isotopen ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ^{99m}Tc, ¹¹¹In, ¹³⁵Sm, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi und ²²⁵Ac ausgewählt ist.

Das erfindungsgemäße Pharmakon kann für die diagnostische Bildgebung mittels Magnetresonanztomographie (Kernspintomographie) oder optical imaging hergerichtet werden. Eine solche Herrichtung kann das Bereitstellen des Pharmakons in einem Kit, umfassend das Pharmakon sowie eine Gebrauchsanweisung, umfassen.

Auf DOTA^{ZOL} basierende Pharmaka des Typs [M]DOTA^{ZOL} sowie davon abgeleitete Derivate (z.B. DOTAM-basierte Derivate) zeigen in *in vitro*-Assays eine Affinität zu Hydroxyapatit, welche die Affinität von bekannten, dreiwertige metallische Radioisotope M enthaltenden Radiopharmaka bei weitem übertrifft.

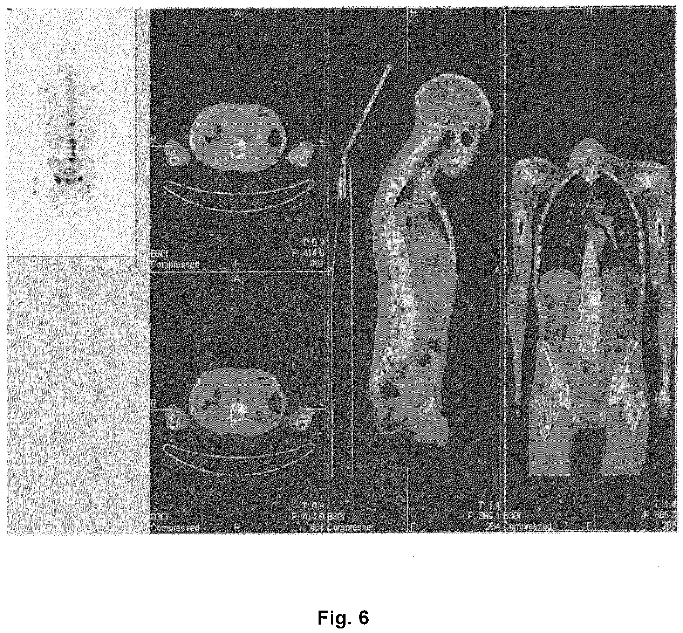

Radiopharmaka des Typs [M]DOTA^{ZOL} sowie davon abgeleitete Derivate (z.B. DOTAM-basierte Derivate) zeigen in *in vivo*-Studien eine wesentlich bessere Bindung an Knochen als die bekannten, dreiwertige metallische Radioisotope M enthaltenden Radiopharmaka. Zudem zeichnen sich [M]DOTA^{ZOL} und dessen Derivate (z.B. DOTAM-basierte Derivate) durch ein günstigstes Anreicherungsverhältnis von Knochen-zu-Blut und Knochen-zu-Weichteilgewebe aus. Dies ist z.B. in Fig. 6 veranschaulicht.

Radiopharmaka des Typs [M]DOTA^{ZOL}, sowie davon abgeleitete Derivate (z.B. DOTAM-basierte Derivate), zeigen im Vergleich zu ²²³RaCl₂ (Xofigo®) keine Akkumulation im Darm und werden schnell über die Nieren ausgeschieden.

Radiopharmaka des Typs [M]DOTA^{ZOL} sowie davon abgeleitete Derivate (z.B. DOTAM-basierte Derivate) zeigen im Vergleich zu aktuell besonders erfolgreichen PSMA-Tracern (PSMA = Prostate Specific Membrane Antigen) eine deutlich intensivere Anreicherung an Knochenmetastasen am gleichen Patienten (Faktoren 2 bis 8) bei gleichzeitig signifikant erniedrigter Akkumulation an gesunden Organen (siehe Beispiel 9). Dementsprechend sind in der bildgebenden Diagnostik-Radiopharmaka der Klasse [⁶⁸Ga]DOTA^{ZOL} (sowie davon abgeleitete Derivate, z.B. DOTAM-basierte Derivate, wie [⁶⁸Ga]DOTAM^{ZOL}) den bekannten Tracern überlegen.

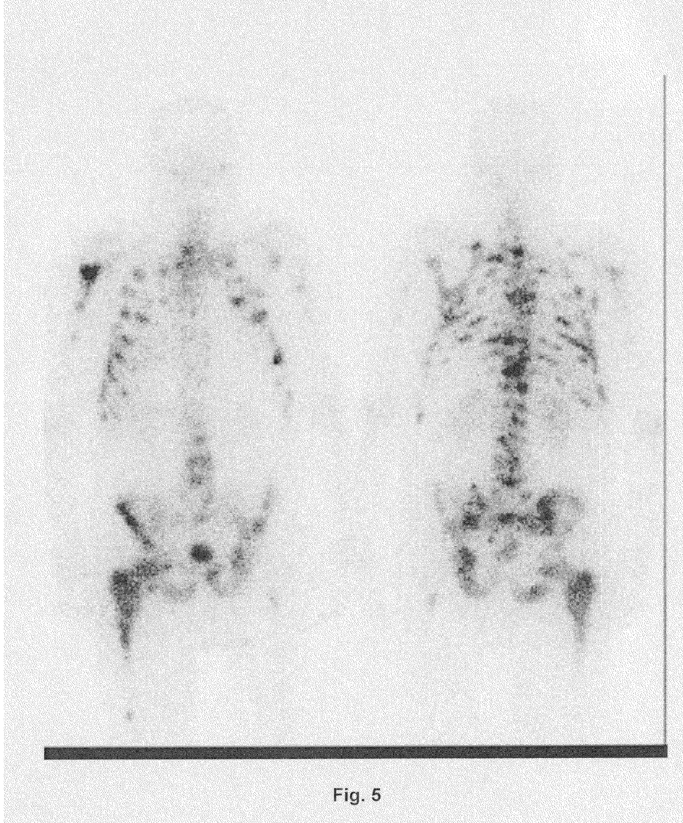

Analog hierzu sind Radiopharmaka der Klasse [¹⁷⁷Lu]DOTA^{ZOL} (sowie davon abgeleitete Derivate, z.B. DOTAM-basierte Derivate, wie [¹⁷⁷Lu]DOTAM^{ZOL}) in ihrer therapeutischen Wirkung den bekannten Tracern auf Basis dreiwertiger metallischer Radioisotope überlegen. Erste klinische Studien beweisen die hohe Selektivität der Verbindung [¹⁷⁷Lu]DOTA^{ZOL}, siehe Fig. 5.

Aufgrund ihrer effektiven und selektiven Anreicherung an Knochentumore sowie ihrer therapeutisch insignifikanten Verweilzeit in allen anderen Organen eröffnen Radiopharmaka der Klasse [¹⁷⁷Lu]DOTA^{ZOL} (sowie davon abgeleitete Derivate, z.B. DOTAM-basierte Derivate, wie [¹⁷⁷Lu]DOTAM^{ZOL}) eine Alternative zur aufwendigen Therapie von Knochentumoren mit ²²³Ra. Die Behandlung von Knochentumoren mit ²²³Ra weist beträchtliche Nachteile auf, wie
(i) beschränkte therapeutische Dosis und Effizienz wegen der hämatotoxischen Wirkung und Akkumulationen von ²²³Ra in Darm, Milz und Leber;
(ii) problematische Handhabung und Dosisbemessung sowie aufwendige Sicherheitsvorkehrungen für den Umgang mit ²²³Ra; und
(iii) chemische Verunreinigung durch ²²⁵Ac, welche zusätzliche kostenintensive Reinigungsschritte erfordert und die Anwendung von ²²³Ra erheblich erschwert.

Darüber hinaus bietet sich ¹⁷⁷Lu-markiertes DOTA^{ZOL} (sowie davon abgeleitete Derivate, z.B. DOTAM-basierte Derivate, wie [¹⁷⁷Lu]DOTAM^{ZOL}) an für eine deutlich potentere Behandlung von Knochenmetastasen nach Prostata-Erkrankung als die derzeit intensiv diskutierten ¹⁷⁷Lu-PSMA-Derivate. Im Vergleich zu diesen ist eine deutlich intensivere Anreicherung an Knochenmetastasen am gleichen Patienten um Faktoren von größer 2 - und damit therapeutisch überlegene Dosimetrie - bei gleichzeitig signifikant erniedrigter Akkumulation - und damit geringerer Körperbelastung - an gesunden Organen zu erwarten.

### Beispiele

Die folgenden Beispiele veranschaulichen Ausführungsformen und Elemente der vorliegenden Erfindung. Wenn in den Beispielen DOTA oder dessen Umsetzung oder Verwendung beschrieben wird, so ist alternativ - statt des Chelators DOTA - der Chelator DOTAM (1,4,7,10-Tetrakis(carbamoylmethyl)-1,4,7,10-tetraazacyclododecan) einsetzbar.

### Beispiel 1: Synthese der Verbindung DOTA^{ZOL}

### Verwendete Instrumente und Chemikalien

ESI-MS: Agilent Technologies 6130 Quadrupole LC/MS Spektrometer oder Finnigan MAT-95 Spektrometer. NMR-Spektrometer: Bruker 600 (Bruker BioSpin AG, Fällanden, Schweiz). DC bzw. Radio-DC: Merck Silica auf Aluminiumfolie, Laufmittel: 0,1 M Citrat pH = 4 oder Acetylaceton:Aceton: konz. HCl (10:10:1). Detektor: Canberra Packard Instant Imager. Radio-HPLC: Waters-system 1525, Säule: MultoKrom (CS-Chromatographie) RP18, 5µ, 250 x 4 mm. Laufmittel: A(10 mM Tetrabutylammonium Citrat pH = 4,5), B(Acetonitril). Gradient 1 mL/min 70(A):30(B) auf 20(A):80(B). Detektor: Berthold Technologies (Dresden). ⁶⁸Ga/⁶⁸Ge-Generator: Eckert & Ziegler AG (Berlin). ¹⁷⁷Lu(III) in 0,05 M HCl: itm AG (München). µPET: Siemens Focus 120. PET-Daten wurden mit Pmod-Software und OSEM 2D Rekonstruktion bearbeitet. Die Radioaktivität in Gewebeproben wurde zerfallskorrigiert mit einem Auto-Gamma-Counter (WIZARD2, Perkin Eimer, Deutschland).

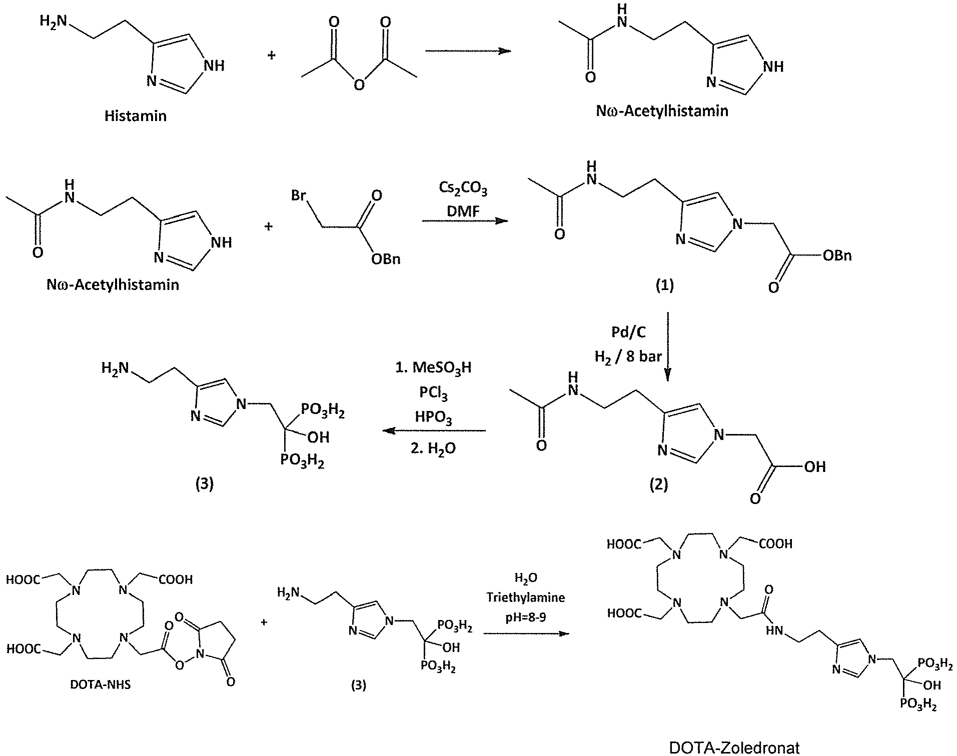

Aus Histamin wird mit Hilfe von Essigsäureanhydrid das primäre N-ω-Acetylhistamin als Ausgangsverbindung dargestellt. Die Verbindung ist kommerziell (Sigma-Aldrich) erhältlich, kann aber auch nach bekannter Literaturvorschrift von van der Merwe et al. Hoppe-Seyler's Zeitschrift für Physiologische Chemie, 177, 1928, 305 synthetisiert werden. DOTA-NHS-ester ist ebenfalls kommerziell erhältlich, können aber auch nach folgender Literaturvorschrift von Rasaneh et al. Nucl. Med. Biol., 36, 2009, 363-369 synthetisiert werden.

Alternativ ist - statt des Chelators DOTA - der Chelator DOTAM (1,4,7,10-Tetrakis(carbamoylmethyl)-1,4,7,10-tetraazacyclododecan) einsetzbar, der ebenfalls kommerziell erhältlich ist oder entsprechend synthetisiert werden kann.

### 1-(Benzylacetat) 4-(Ethylacetamid)-Imidazol (1)

N-ω-Acetylhistamin 1 g (6,53 mmol) wird in 50 mL trockenem DMF gelöst und 4,4 g (13 mmol) Cäsiumcarbonat zugegeben. Die Lösung wird unter Argon-Atmosphäre und Eiskühlung gerührt. 2,2 g (13 mmol) Benzylbromoacetat werden gelöst in 50 mL trockenem DMF langsam zur Suspension zu getropft. Die Mischung wird 12 Stunden gerührt bevor Aktivkohle zugegeben wird. Anschließend werden die Feststoffe abfiltriert und das Lösungsmittel im Vakuum entfernt. Das Rohprodukt wird aus Acetylacetat umkristallisiert und 1,14 g (58%) des 1-(Benzylacetat)-4-(Ethylacetamid)-Imidazol (1) als schwach gelben Feststoff erhalten.
¹H-NMR (CDCl₃, 300 MHz): δ 1,97 (s, 3H, CH₃-CO), 2,76 (t, JH = 6,3 Hz, 2H, CH₂-CH₂), 3,53 (q, JH = 6,0 Hz, 2H, CH₂-CH₂), 4,70 (s, 2H, N-CH₂-CO), 5,22 (s, 2H, Bn-CH₂-CO), 6,53 (bs, 1H, NH), 6,75 (d, JH = 1,3 Hz, Imidazol-H), 7,39 (m, 5H, Benzyl), 7,44 (d, JH = 1,3 Hz, ¹H, Imidazol-H). FD-MS(+): cald 301,14 obsd 302,3 (M + H⁺), 603,2 (2M + H⁺).

### 1-(1-Hydroxy-Ethan-1,1-Bis(Phosphonsäure))4-(Ethylamin)-Imidazol (3)

300 mg (1 mmol) von (1) werden in 20 mL trockenem Methanol gelöst und Pd/C (10%w) zugegeben. Die Suspension unter Wasserstoffatmosphäre (5 bar) für 12 Stunden gerührt. Nach der Entfernung des Lösungsmittels und der Feststoffe wurde die entschützte Säure (2) (208 mg, 98%) direkt weiter umgesetzt. 1 mL Methansulfonsäure und 164 mg (2 eq.) phosphorige Säure wurden unter Rühren zugegeben. Die Mischung wurde auf 75°C erhitzt und 300 mg (2,2 eq.) Phosphortrichlorid unter Schutzgasamtosphäre langsam zugetropft. Nach 12 Stunden wurde die Reaktionsmischung auf Raumtemperatur abgekühlt und mit 2 mL Eiswasser versetzt. Anschließend wurde die Lösung für 24 Stunden unter Rückfluss erhitzt. Nach der Zugabe von Aktivkohle wurden alle Feststoffe abfiltriert und konzentrierte Natriumhydroxid-Lösung zugetropft, bis ein weißer Feststoff beginnt auszufällen. Zur Komplettierung der Fällung wurde die Suspension 24 Stunden bei 4°C gelagert. Im finalen Schritt wird der erhaltene Feststoff aus kochendem Wasser umkristallisiert und 88,6 mg (28%) vom oben genannten Hydroxybisphosphonat (3) erhalten.
¹H-NMR (D₂O/NaOD, 300 MHz): δ 2,46 (m, 2H, CH₂-CH₂), 2,66 (m, 2H, CH₂-CH₂), 4,28 (m, 2H, N-CH₂-Phosponat), 6,89 (s, 1H, Imidazol-H), 7,54 (s, 1H, Imidazol-H). ³¹P-NMR (D₂O/NaOD, 162,05 MHz): δ 14,4. ESI-MS(+): cald 315,04 obsd 316,05 (M + H⁺), 338,04 (M + Na⁺).

### DOTA^{ZOL}

15,75 mg (0,05 mmol) (3) werden in 1 mL Wasser suspendiert und Triethylamin (TEA) zugegeben, bis sich alle Feststoffe gelöst haben. 38 mg (0,05 mmol) DOTA-NHS-Ester gelöst in 0,5 mL Wasser werden langsam zur Bisphosphonat-Lösung zugetropft. Die Reaktionsmischung wird bei 50°C für 24 Stunden gerührt. Der pH-Wert wird regelmäßig kontrolliert und durch Zugabe von TEA zwischen 8 und 9 gehalten. Das Rohprodukt wird durch preparative HPLC (Phenomenex Synergy Hydro-RP 80, 10 µ, 250 x 30 mm, Laufmittel: H₂O + 0,1% TFA) von den Edukten getrennt. Im zweiten Schritt wird das Rohprodukt durch Festphasenextraktion weiter aufgereinigt (NH₂-Phase, Merck LiChroprep NH₂). Das Produkt wird nach Waschen der Festphase mit Wasser/Methanol/Wasser durch eine Lösung aus H₂O + 2% TFA von der Festphase eluiert. Nach Lyophilierung erhält man 5,6 mg (15,7%) eines weißen Feststoffs.
¹H-NMR (D₂O/NaOD, 300 MHz): δ 2,42 (m, 2H, CH2-CH2), 2,61 (m, 2H, CH₂-CH₂), 2,9-3,5 (b, 16H, cyclen-CH₂), 3,75 (bs, 8H, -CH₂-CO), 4,55 (m, 2H, N-CH₂-Phosponat), 7,28 (s, 1H, Imidazole-H), 8,54 (s, 1H, Imidazol-H). ³¹P-NMR (D₂O/NaOD, 162,05 MHz): δ 14,3. ESI-MS(+): cald 701,2 obsd 702,5 (M + H⁺), 351,1 (M + 2H⁺).

Alternativ ist statt des Chelators DOTA der Chelator DOTAM (1,4,7,10-Tetrakis(carbamoylmethyl)-1,4,7,10-tetraazacyclododecan) einsetzbar.

### Beispiel 2: Synthese von [⁶⁸Ga]DOTA^{ZOL}

25 nmol DOTA^{ZOL} werden in 500 µL Natriumacetat-Puffer (0.5 M, pH = 4) gelöst und mit 400 µL ⁶⁸Ga(III) Lösung versetzt. Die Mischung wird 15 min bei 98°C erhitzt. Anschließend wird die Reaktionslösung steril filtriert. Die radiochemische Reinheit wurde durch Dünnschichtchromatographie und HPLC auf größer/gleich 95% bestimmt.

### Beispiel 3: Synthese von [¹⁷⁷Lu]DOTA^{ZOL}

10 nmol DOTA^{ZOL} pro 1 GBq ¹⁷⁷Lu(III) werden in 1 mL Natriumacetat-Puffer (0.1 M, pH = 5.0) gelöst und mit ¹⁷⁷Lu(III) -Lösung versetzt. Die Mischung wird 30 min bei 98°C erhitzt. Anschließend wird die Reaktionslösung steril filtriert. Die radiochemische Reinheit wurde durch Dünnschichtchromatographie und HPLC auf größer/gleich 98% bestimmt.

### Beispiel 4: NHS-Kupplung von DOTAGA in leicht basischer wässriger Lösung

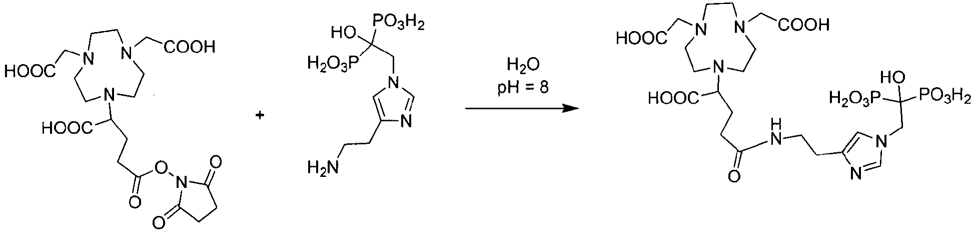

### Beispiel 5: NCS-Kupplung eines HEHA-Derivats in leicht basischer wässriger Lösung

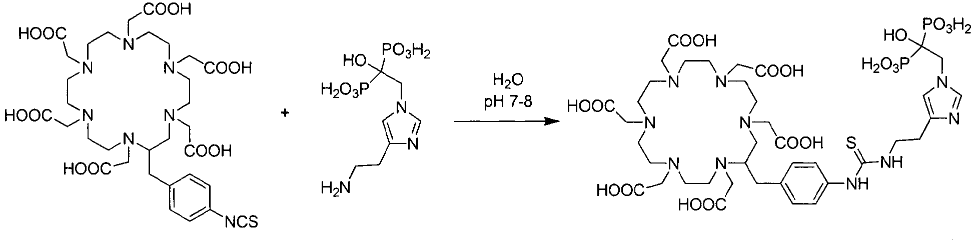

### Beispiel 6: Quadratsäure-Kupplung eines DO3A-Derivats in leicht basischer wässriger Lösung.

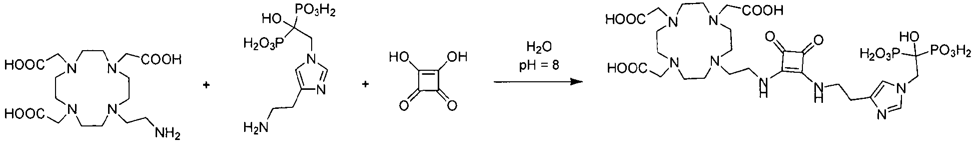

### Beispiel 7: Mannich-Reaktion von DO3A in saurer Lösung

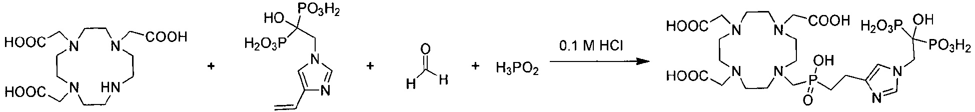

### Beispiel 8: In vivo-Experimente in Ratten

Gesunden Wistar Ratten (N = 5) mit einem Gewicht zwischen 140 bis 220 g wurde unter Isofluran Anästhesie 15-18 MBq der ⁶⁸Ga-markierten Verbindung bzw. der ¹⁷⁷Lumarkierten Verbindungen verdünnt in isotoner Kochsalzlösung in die Schwanzvene appliziert. Die Ratten wurden 60 min nach Injektion getötet, Organproben entnommen, gewogen und die Akkumulation des markierten Bisphosphonats im Gewebe in SUV (standardized uptake value) bestimmt, nach der Formel: SUV = (Aktivität pro g Gewebe)/(injizierte Aktivität) x Körpergewicht.

Als Vergleichssubstanzen wurden das bekannte α-Hydroxy-BP BPAPD und ein Pamidronat-DOTA-Konjugat (DOTA^{PAM}) gewählt. Diese Verbindungen repräsentieren die vorstehend erwähnten, im Stand der Technik bekannten Verbindungen, welche ihre affine Aminofunktion offenbar durch die Derivatisierung mit dem bifunktionellen Chelator verloren haben.

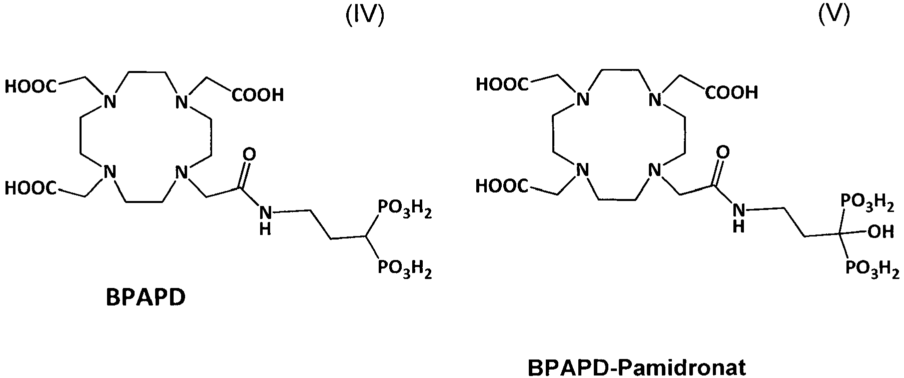

Messergebnisse zur Organdistribution der vorstehend beschriebenen ⁶⁸Ga-markierten Bisphosphonate sind in den nachfolgenden Tabellen 1 und 2 zusammengestellt.

**Tabelle 1: Ex vivo-Biodistribution von [⁶⁸Ga]BPAPD, [⁶⁸Ga]DOTA^{PAM} und [⁶⁸Ga]DOTA^{ZOL} in Wistar Ratten nach 60 min. Daten dargestellt in SUV (Standardabweichung) aus fünf Tieren. ^{†}P < 0,05 vs. [⁶⁸Ga]BPAPD; ^{‡}P < 0,05 vs. [⁶⁸Ga]DOTA^{PAM}.**

| **SUV** | | | |
|---|---|---|---|
| Organ | [⁶⁸Ga]BPAPD | [⁶⁸Ga]DOTA^{PAM} | [⁶⁸Ga]DOTA^{ZOL} |
| Lunge | 0,43 (0,08) | 0,53 (0,16) | 0,45 (0,11) |
| Leber | 0,37 (0,11) | 0,43 (0,04) | 0,28 (0,03)^{‡} |
| Milz | 0,23 (0,08) | 0,31 (0,04) | 0,17 (0,02)^{‡} |
| Nieren | 0,56 (0,08) | 0,48 (0,06) | 0,53 (0,04) |
| Muskel | 0,17 (0,02) | 0,09 (0,02) | 0,08 (0,02)^{†} |
| Herz | 0,32 (0,09) | 0,23 (0,02) | 0,14 (0,04)^{†‡} |
| **Blut** | **0,86 (0,21)** | **0,60 (0,03)** | **0,47 (0,19)^{†}** |
| Darm | 0,26 (0,05) | 0,28 (0,12) | 0,14 (0,08) |
| **Femur** | **3,21 (0,29)** | **4,53 (0,17)** | **5,40 (0,62)^{†‡}** |

**Tabelle 2: Knochen/Organ-Verhältnisse von [⁶⁸Ga]BPAPD, [⁶⁸Ga]DOTA^{PAM} und [⁶⁸Ga]DOTA^{ZOL} in Wistar Ratten nach 60 min.**

| **Knochen-zu-Organ-Verhältnisse** | | | |
|---|---|---|---|
| | [⁶⁸Ga]BPAPD | [⁶⁸Ga]DOTA^{PAM} | [⁶⁸Ga]DOTA^{ZOL} |
| Lunge | 7,47 | 8,61 | 12,06 |
| Leber | 8,68 | 10,02 | 19,21 |
| Milz | 13,96 | 14,51 | 31,71 |
| Nieren | 5,73 | 9,44 | 10,22 |
| Muskel | 18,88 | 47,96 | 65,96 |
| Herz | 10,03 | 19,49 | 37,97 |
| **Blut** | **3,73** | **7,61** | **11,47** |
| Darm | 12,35 | 16,11 | 38,67 |

**Tabelle 3: Pharmakologische Parameter (2-Compartment-Modell) aus den in vivo µPET-Experimenten mit [⁶⁸Ga]BPAPD, [⁶⁸Ga]DOTA^{PAM} und [⁶⁸Ga]DOTA^{ZOL} in Wistar-Ratten.**

| Eliminierungs Halbwertszeit | [⁶⁸Ga]BPAPD | [⁶⁸Ga]DOTA^{PAM} | [⁶⁸Ga]DOTA^{ZOL} |
|---|---|---|---|
| t_{1/2}(α) | 5 min | 4,5 min | 5 min |
| **t_{1/2}(β)** | **4 h** | **4,5 h** | **2,3 h** |

Alternativ ist statt des Chelators DOTA der Chelator DOTAM (1,4,7,10-Tetrakis(carbamoylmethyl)-1,4,7,10-tetraazacyclododecan) einsetzbar.

### Beispiel 9: Anreicherung an Knochenmetastasen

Radiopharmaka des Typs [M]DOTA^{ZOL} sowie davon abgeleitete Derivate (z.B. DOTAM-basierte Derivate) zeigen im Vergleich zu aktuell besonders erfolgreichen PSMA-Tracern (PSMA = Prostate Specific Membrane Antigen) eine deutlich intensivere Anreicherung an Knochenmetastasen am gleichen Patienten (Faktoren 2 bis 8) bei gleichzeitig signifikant erniedrigter Akkumulation an gesunden Organen (siehe Beispiel 9). Die Tabelle zeigt die gemessene Aufnahme (uptake), (als SUV-max-Werte) von [⁶⁸Ga]DOTA^{ZOL} und [⁶⁸Ga]HBED-PSMA^{CC} im direkten Vergleich an einem Patienten mit Prostatakarzinom und Knochenmetastasen.

**Tabelle 4: SUV-max-Werte von [⁶⁸Ga]DOTA^{ZOL} und [⁶⁸Ga]HBED-PSMA^{CC} im direkten Vergleich an einem Patienten mit Prostatakarzinom und Knochenmetastasen.**

| | **Organ** | **[⁶⁸Ga]DOTA^{ZOL} PET/CT** | **[⁶⁸Ga]HBED-PSMA^{CC}** PET/CT |
|---|---|---|---|
| | | Tag n | Tag n+2 (d.h. 2 Tage später) |
| | | **SUV-max** | |
| Knochenläsionen | C4 Wirbelkörper und Hüftsiel | 12,38 | 7,97 |
| | 8. rechte hintere Rippe | 7,99 | 7,12 |
| | T9 Wirbelkörper | 17,08 | 7,60 |
| | T11 Wirbelkörper | 32,29 | 12,98 |
| | L2 Wirbelkörper | 68,92 | 8,85 |
| | L3 Wirbelkörper | 62,96 | 9,46 |
| | L4 Wirbelkörper | 34,51 | 10,98 |
| | L5 Wirbelkörper | 21,94 | 19,99 |
| | Kreuzbein | 17,98 | 14,96 |
| | Rechtes Sitzbein | 32,55 | 14,96 |
| | Linker Knochen, Höhe Sitzbein- und Schambeinkochen | 62,32 | 8,99 |
| | Rechte Hüftpfanne | 25,39 | 11,71 |
| Weichgewebe | Rechte Ohrspreicheldrüse | 1,22 | 5,80 |
| | Linke Ohrspreicheldrüse | 1,46 | 6,48 |
| | Leber | 2,85 | 6,48 |
| | Milz | 4,50 | 3,27 |
| | Rechte Niere | 9,34 | 22,72 |
| | Linke Niere | 4,12 | 5,75 |
| | Blase | 15,53 | 22,23 |

### Kurze Beschreibung der Figuren

Die beigefügten Figuren veranschaulichen Ausführungsformen und Elemente der vorliegenden Erfindung, schränken den Gegenstand der Erfindung aber nicht auf die in den Figuren veranschaulichten Ausführungsformen und Elemente ein. Wenn in den Figuren DOTA oder dessen Umsetzung oder Verwendung beschrieben wird, so ist alternativ - statt des Chelators DOTA - der Chelator DOTAM (1,4,7,10-Tetrakis(carbamoylmethyl)-1,4,7,10-tetraazacyclododecan) einsetzbar.

**Fig 1****:** Vergleich von [^{99m}Tc]MDP und ²²³RaCl₂ an Tag 1, 2 und Tag 6 nacph der Injektion: aus O. Sartor, P. Hoskin, Ø. S. Bruland, Targeted radio-nuclide therapy of skeletal metastases, Cancer Treatment Reviews, 2013; 39: 18-26.

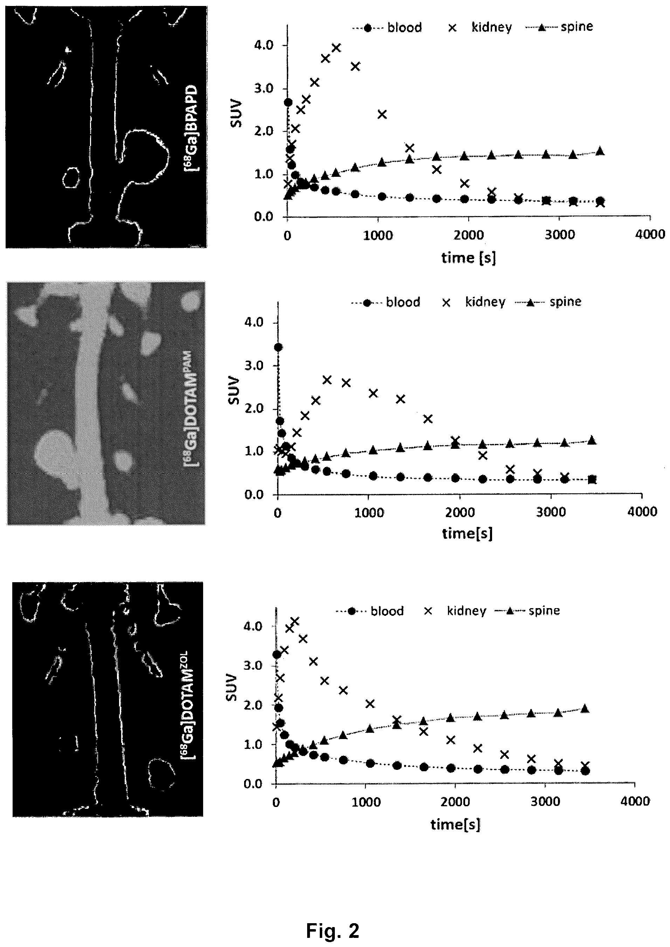

**Fig. 2****:** µPET-Aufnahmen verschiedener ⁶⁸Ga(III) markierter makrozyklischer Bisphosphonate in gesunden Wistar-Ratten nach 60 min im Betriebsmodus Maximum Intesity Projection.

**Fig. 3** zeigt schematisch eine erste Vorrichtung 1 zur Herstellung eines Radiopharmakons aus der erfindungsgemäßen Verbindung V und einem metallischen Radioisotop, wie oben detailliert beschrieben.

**Fig. 4** zeigt eine zweite Vorrichtung 2 zur Herstellung eines Radiopharmakons aus der erfindungsgemäßen Verbindung V und einem metallischen Radioisotop M, wie oben detailliert beschrieben.

**Fig. 5****:** Verteilung von [¹⁷⁷Lu]DOTA^{ZOL} im Patienten mit disseminierten Knochenmetastasen: [¹⁷⁷Lu]DOTA^{ZOL}-Szintigraphie an einem Patienten mit Prostatakarzinom 6 Stunden nach Injektion. In einer ersten therapeutischen Anwendungen konnte der PSA-Wert, welcher ein wichtiger Marker in der Verlaufskontrolle des Prostatakarzinoms darstellt, innerhalb von zwei Monaten von anfänglich 478 ng/mL auf 88 ng/mL nach nur einer Behandlung mit 5,5 GBq [¹⁷⁷Lu]DOTA^{ZOL} gesenkt werden. Äquivalent kann ¹⁷⁷Lu-DOTAM^{ZOL} verwendet werden.

**Fig. 6****:** PET/CT-Scan eines Prostatakarzinom-Patienten mit Knochenmetastasen, untersucht mit ⁶⁸Ga-DOTA^{ZOL}. Äquivalent kann ⁶⁸Ga-DOTAM^{ZOL} verwendet werden.

## Patentansprüche

1. Verbindung V zum Komplexieren von metallischen Isotopen, umfassend einen Chelator X und einen oder mehrere, mit dem Chelator X konjugierte Targetingvektoren mit der Struktur -L₁-R₁-L₂-R₂-L₃-R₃ , wobei
L₁ aus der Gruppe, umfassend Amid, Phosphinat, Alkyl, Triazol, Thioharnstoff, Ethylen, Maleimid, -(CH₂)ₖ- und -(CH₂CH₂O)ₖ- mit k = 1, 2, 3, 4, 5, 6, 7, 8, 9 oder 10 gewählt ist,
L₂ aus -(CH₂)ₘ- und -(CH₂CH₂O)ₘ- mit m = 1, 2, 3, 4, 5, 6, 7, 8, 9 oder 10 gewählt ist, und
L₃ aus -(CH₂)ₙ- und -(CH₂CH₂O)ₙ- mit n = 1, 2, 3, 4, 5, 6, 7, 8, 9 oder 10 gewählt ist,
**dadurch gekennzeichnet, dass** 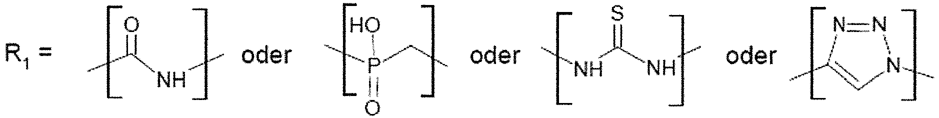 R2 gewählt ist aus der Gruppe, umfassend eine Restgruppe eines:
Pyrrols, Pyridins, Pyrimidins, Furans, Azols, Triazols, Tetrazols, Pyrazols, Imidazols, Oxazols, Oxadiazols, Thiophens, Thiazols, Thiadiazols, Azins, Oxazins, Thiazins, Naphthalins, Chinolins, Chromens oder Thiochromens;
und

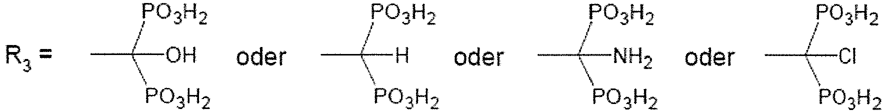

2. Verbindung V nach Anspruch 1, **dadurch gekennzeichnet, dass** der Chelator X gewählt ist aus der Gruppe, umfassend EDTA (Ethylendiamintetraacetat), EDTMP (Diethylentriaminpenta(methylenphosphonsäure)), DTPA (Diethylentriaminpentaessigsäure) und dessen Derivate, DOTA (1,4,7,10-Tetraazacyclododecan-1,4,7,10-tetraessigsäure), DOTAGA (Dodeca-1-glutarsäure-1,4,7,10-tetraamin-triessig-säure), DOTAM (1,4,7,10-Tetrakis(carbamoylmethyl)-1,4,7,10-tetraazacyclododecan) und anderen DOTA-Derivaten, TRITA (Trideca-1,4,7,10-tetraamin-tetraessigsäure), TETA (Tetradeca-1,4,8,11-tetraamin-tetraessigsäure) und dessen Derivate, NOTA (Nona-1,4,7-triamin-triessigsäure) und dessen Derivate wie beispielsweise NOTAGA (1,4,7-triazacyclononan,1-glutarsäure,4,7-essigsäure), NOPO (1,4,7-triazacyclononan-1,4-bis[methylen(hydroxymethyl)phosphinsäure]-7-[methylen(2-carboxyethyl)phosphinsäure]) und dessen Derivate, PEPA (Pentadeca-1,4,7,10,13-pentaaminpentaessigsäure) und dessen Derivate, HEHA (Hexadeca-1,4,7,10,13,16-hexaamin-hexaessigsäure) und dessen Derivate, HBED (Hydroxybenzyl-ethylen-diamin) und dessen Derivate, DEDPA und dessen Derivate, wie H₂DEDPA (1,2-[{6-(carboxylat-)pyridin-2-yl}methylamin]ethan), DFO (Deferoxamin) und dessen Derivate, Deferipron, CP256 (4-Acetylamino-4-{2-[(3-hydroxy-1,6-dimethyl-4-oxo-1,4-dihydro-pyridin-2-ylmethyl)-carbamoyl]-ethyl}-heptandisäure bis-[(3-hydroxy-1,6-dimethyl-4-oxo-1,4-dihydro-pyridin-2-ylmethyl)-amid]) und dessen Derivate, wie YM103; TRAP (Triazacyclononan-phosphinsäure) und dessen Derivate, TEAP (Tetraazycyclodecan-phosphinsäure) und dessen Derivate, AAZTA (6-Amino-6-methylperhydro-1,4-diazepin-N,N,N',N'-tetraessigsäure) und Derivate wie DATA; und SarAr (1-N-(4-aminobenzyl)-3,6,10,13,16,19-hexaazabicyclo[6.6.6]-eicosan-1,8-diamin) und Salze davon.

3. Verbindung V nach Anspruch 1 oder 2, **dadurch gekennzeichnet, dass** die Verbindung V eine Struktur gemäß der Formel I aufweist 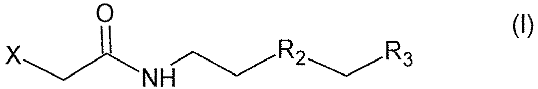 worin X den Chelator bezeichnet
oder
dass die Verbindung V eine Struktur der Formel DOTAGA-L₁-R₁-L₂-R₂-L₃-R₃ aufweist:

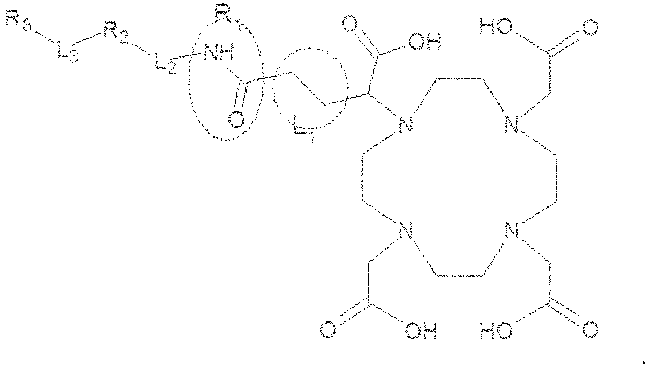

4. Verbindung V nach Anspruch 1, 2 oder 3, **dadurch gekennzeichnet, dass** die Verbindung V eine der folgenden Strukturen aufweist: 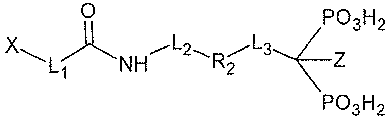 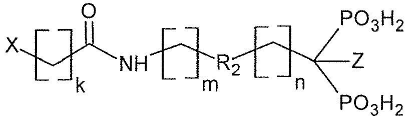 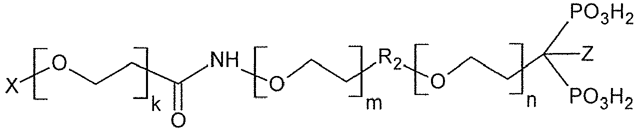 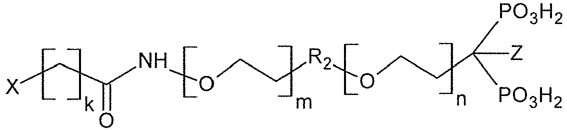 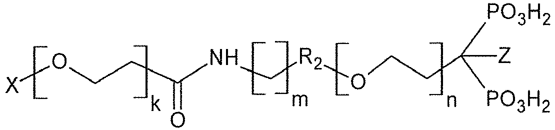 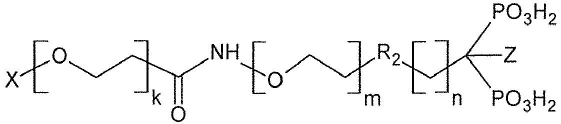 mit
k = 1, 2, 3, 4, 5, 6, 7, 8, 9 oder 10;
m = 1, 2, 3, 4, 5, 6, 7, 8, 9 oder 10;
n = 1, 2, 3, 4, 5, 6, 7, 8, 9 oder 10; und
Z = OH, H, NH₂ oder Cl.

5. Verbindung V nach Anspruch 1, 2, 3 oder 4, **dadurch gekennzeichnet, dass** R₂ ausgewählt ist aus der Gruppe, umfassend eine Restgruppe aus

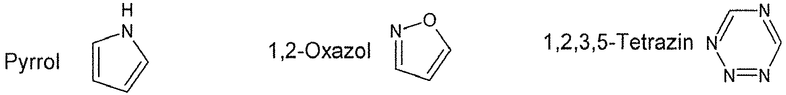

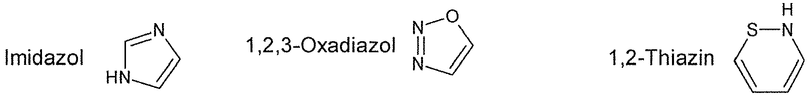

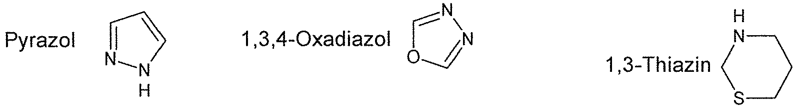

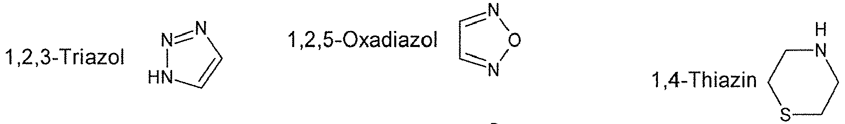

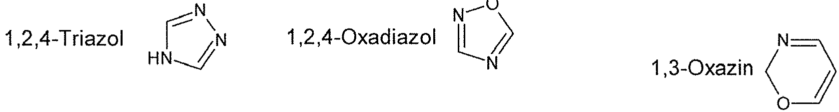

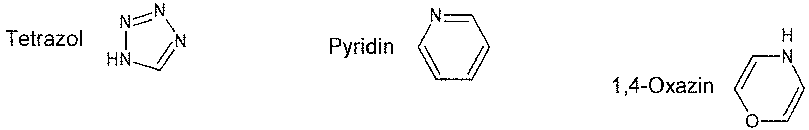

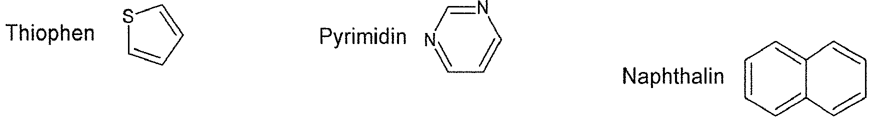

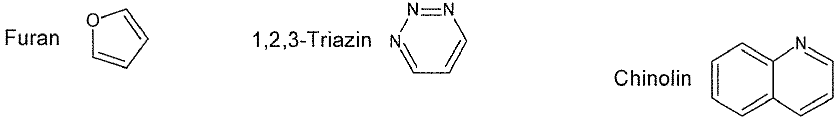

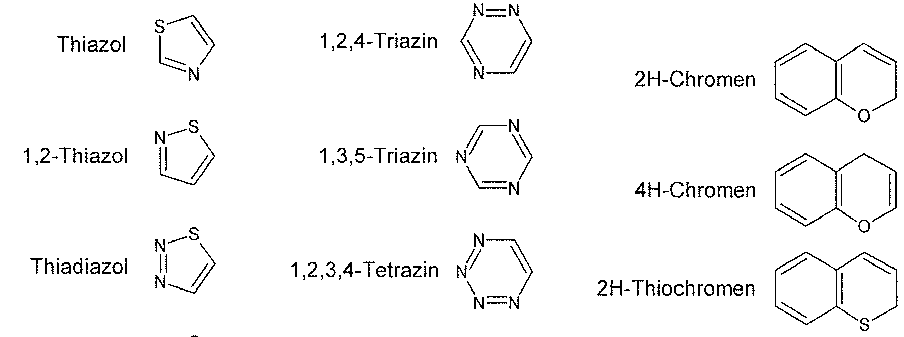

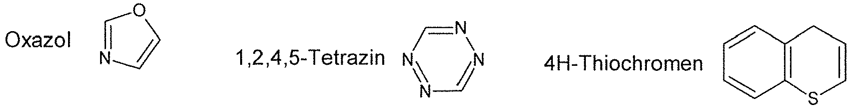

6. Verbindung V nach einem der vorgenannten Ansprüche 1, 2, 3 oder 4, **dadurch gekennzeichnet, dass** die Verbindung V eine Struktur gemäß Formel II aufweist: 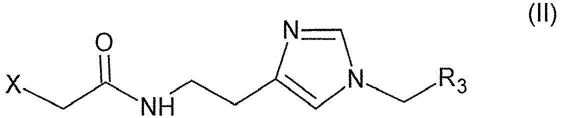 worin X jeweils den Chelator bezeichnet.

7. Verbindung V nach einem der vorgenannten Ansprüche, **dadurch gekennzeichnet, dass** die Verbindung V
(i) 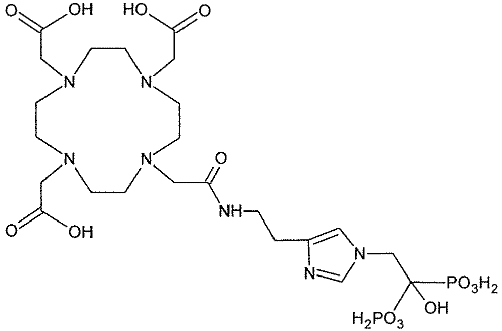 oder
(ii) ein Derivat von (i), wobei das Derivat L₁, L₂, L₃, R₁, R₂, R₃, wie bei (i) definiert, aufweist und der Chelator X DOTAM ist,
ist.

8. Pharmakon bestehend aus der Verbindung V nach Anspruch 1, 2, 3, 4, 5, 6 oder 7 und einem mit der Verbindung V komplexierten, metallischen Isotop M.

9. Pharmakon nach Anspruch 8, **dadurch gekennzeichnet, dass** das metallische Isotop M gewählt ist aus der Gruppe, umfassend ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ⁹⁹mTc, ¹¹¹In, ¹³⁵Sm, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi und ²²⁵Ac.

10. Verfahren zur Herstellung eines Pharmakons nach Anspruch 8 oder 9, welches die folgenden Schritte umfasst:
(a) Bereitstellen einer die Verbindung V nach einem der Ansprüche 1 bis 7 enthaltenden Lösung S;
(b) Bereitstellen eines metallischen Isotops M, wie beispielsweise ⁶⁸Ga(III); und
(c) Ligieren des metallischen Isotops M mit der Verbindung V unter Bildung eines Komplexes MV des metallischen Isotops M mit der Verbindung V in einer Lösung F.

11. Verfahren nach Anspruch 10, **dadurch gekennzeichnet, dass** in Schritt (b) das metallische Isotop M in einer Lösung bereit gestellt wird.

12. Verfahren nach Anspruch 10, **dadurch gekennzeichnet, dass** in Schritt (b) ein Radionuklidgenerator mit einem Mutternuklid und einem durch Zerfall des Mutternuklids gebildeten metallischen Isotop M bereitgestellt und in Schritt (c) das metallische Isotop M mit der Lösung S von dem Mutternuklid separiert wird.

13. Verwendung einer Verbindung V nach Anspruch 1, 2, 3, 4, 5, 6 oder 7 als Markierungsvorläufer zur Herstellung eines Pharmakons.

14. Pharmakon nach Anspruch 8 oder 9 zur Verwendung in einem bildgebenden Verfahren mittels Positronen-Emissions-Tomographie oder Einzelphotonen-Emissions-Computertomographie, insbesondere zur Abbildung von pharmakokinetischen Vorgängen, oder zur Verwendung für die diagnostische Bildgebung mittels Magnetresonanztomographie (Kernspintomographie) oder optical imaging.

15. Pharmakon nach Anspruch 8 oder 9 zur Verwendung in einem Behandlungs- oder Therapieverfahren.

16. Pharmakon nach Anspruch 8 oder 9 zur Verwendung in einem Verfahren zur Behandlung von Knochenerkrankungen und Knochentumoren.

17. Pharmakon zur Verwendung nach Anspruch 16 zur Behandlung von Erkrankungen an nicht-manifestierten Knochenmetastasen.

18. Pharmakon zur Verwendung nach Anspruch 17, wobei die Behandlung die Anreicherung in der Tumorzelle umfasst, um die Farnesyl-Pyrophosphat-Synthase (FPPS) zu hemmen.

19. Pharmakon nach Anspruch 8 oder 9 zur Verwendung als Additiv in künstlicher Knochensubstanz, in Knochenzement oder in Knochenimplantaten.

20. Verbindung V nach Anspruch 1, 2, 3, 4, 5, 6 oder 7 in Verbindung mit einem metallischen Isotop M, wie Gadolinium, zur Verwendung bei der Herstellung eines Pharmakons.

21. Verbindung V zur Verwendung nach Anspruch 20, wobei das metallische Isotop M unter den in Anspruch 9 genannten Isotopen ausgewählt ist.

## Claims

1. Compound V for complexing metallic isotopes, comprising a chelator X and one or more targeting vectors conjugated with the chelator X, said targeting vectors having the structure -L₁-R₁-L₂-R₂-L₃-R₃, wherein
L₁ is selected from the group comprising amide, phosphinate, alkyl, triazole, thiourea, ethylene, maleimide, -(CH₂)ₖ- and -(CH₂CH₂O)ₖ-, with k = 1, 2, 3, 4, 5, 6, 7, 8, 9, or 10,
L₂ is selected from -(CH₂)ₘ- and -(CH₂CH₂O)ₘ-, with m = 1, 2, 3, 4, 5, 6, 7, 8, 9, or 10, and
L₃ is selected from -(CH₂)ₙ- and -(CH₂CH₂O)ₙ-, with n = 1, 2, 3, 4, 5, 6, 7, 8, 9, or 10,
**characterized in that** 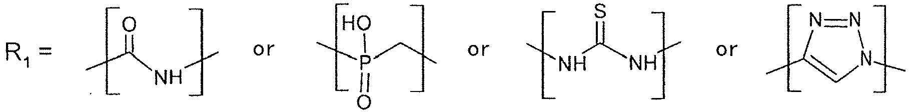 R₂ is selected from the group comprising a substituent group of a:
pyrrole, pyridine, pyrimidine, furan, azole, triazole, tetrazole, pyrazole, imidazole, oxazole, oxadiazole, thiophen, thiazole, thiadiazole, azine, oxazine, thiazine, naphthalene, quinoline, chromene, or thiochromene;
and

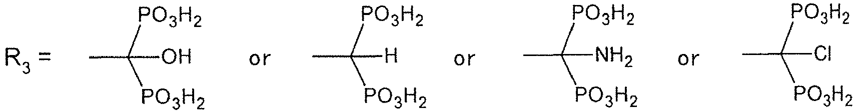

2. Compound V according to claim 1, **characterized in that** the chelator X is selected from the group comprising EDTA (ethylenediamine-tetraacetate), EDTMP (diethylenetriamine penta(methylene phosphonic acid)), DTPA (diethylenetriamine pentaacetic acid) and its derivatives, DOTA (1,4,7,10-tetraazacyclododecane-1,4,7,10-tetraacetic acid), DOTAGA (dodeca-1-glutaric acid-1,4,7,10-tetraamine-triacetic acid), DOTAM (1,4,7,10-tetrakis(carbamoylmethyl)-1,4,7,10-tetraazacyclododecane) and other DOTA derivatives, TRITA (trideca-1,4,7,10-tetraamine tetraacetic acid), TETA (tetradeca-1,4,8,11-tetraamine-tetraacetic acid) and its derivatives, NOTA (nona-1,4,7-triamine-triacetic acid) and its derivatives, e.g., NOTAGA (1,4,7-triazacyclononane,1-glutaric acid,4,7-acetic acid), NOPO (1,4,7-triazacyclononane-1,4-bis[methylene(hydroxymethyl)hypophosphorous acid]-7-[methylene(2-carboxyethyl)hypophosphorous acid]) and its derivatives, PEPA (pentadeca-1,4,7,10,13-pentaamine pentaacetic acid) and its derivatives, HEHA (hexadeca-1,4,7,10,13,16-hexaamine hexaacetic acid) and its derivatives, HBED (hydroxybenzyl-ethylenediamine) and its derivatives, DEDPA and its derivatives, such as H₂DEDPA (1,2-[{6-(carboxylate-)pyridine-2-yl}methylamine]ethane), DFO (deferoxamine) and its derivatives, Deferiprone, CP256 (4-acetylamino-4-{2-[(3-hydroxy-1,6-dimethyl-4-oxo-1,4-dihydro-pyridine-2-ylmethyl)-carbamoyl]-ethyl}-heptane diacid bis-[(3-hydroxy-1,6-dimethyl-4-oxo-1,4-dihydro-pyridine-2-ylmethyl)-amide]) and its derivatives, such as YM103; TRAP (triazacyclononane-hypophosphorous acid) and its derivatives, TEAP (tetraazycyclodecane-hypophosphorous acid) and its derivatives, AAZTA (6-amino-6-methylperhydro-1,4-diazepine-N,N,N',N'-tetraacetic acid) and derivatives such as DATA; and SarAr (1-N-(4-aminobenzyl)-3,6,10,13,16,19-hexaazabicyclo[6.6.6]-eicosane-1,8-diamine) and salts thereof.

3. Compound V according to claim 1 or 2, **characterized in that** the compound V has a structure according to Formula I 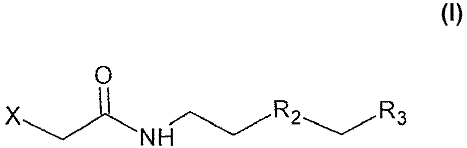 wherein X designates the chelator,
or
**in that** compound V has a structure of formula DOTAGA-L₁-R₁-L₂-R₂-L₃-R₃

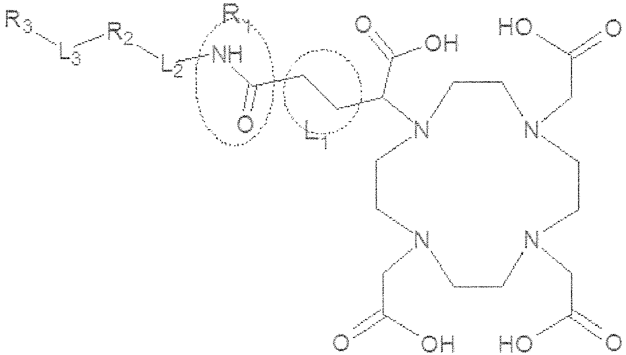

4. Compound V according to claim 1, 2 or 3, **characterized in that** compound V has one of the following structures: 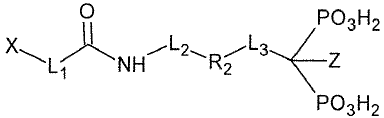 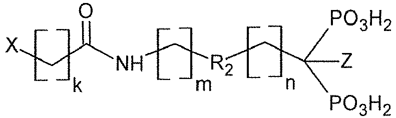 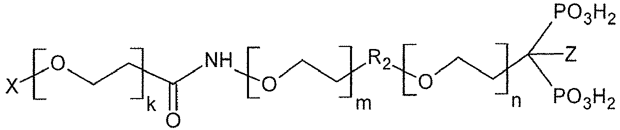 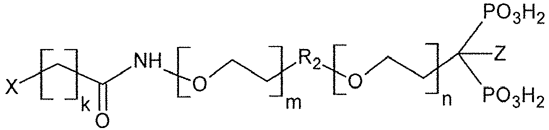 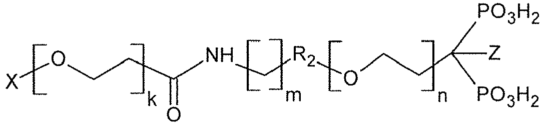 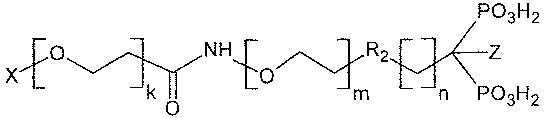 with
k = 1, 2, 3, 4, 5, 6, 7, 8, 9 or 10;
m = 1,2, 3, 4, 5, 6, 7, 8, 9 or 10;
n = 1, 2, 3, 4, 5, 6, 7, 8, 9 or 10; and
Z = OH, H, NH2 or Cl.

5. Compound V according to claim 1, 2, 3 or 4, **characterized in that** R₂ is selected from group, comprising the substituent group of

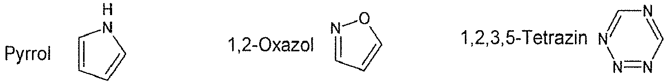

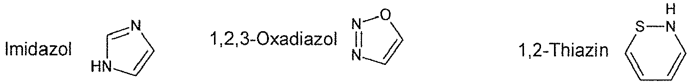

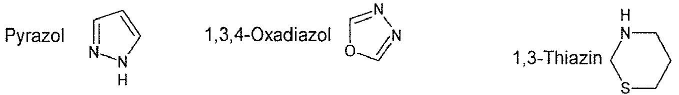

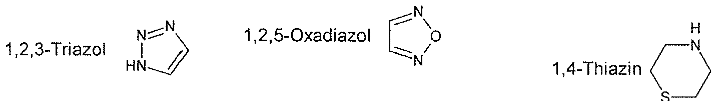

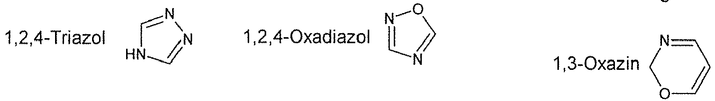

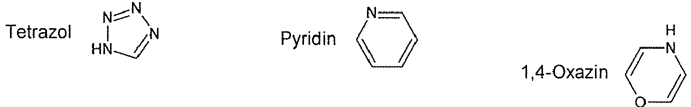

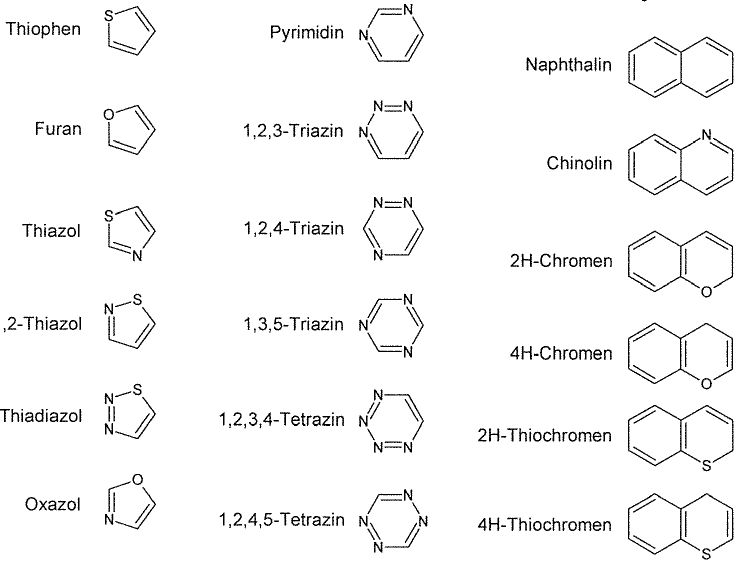

6. Compound V according to claim 1, 2, 3 or 4, **characterized in that** compound V has a structure according to Formula II 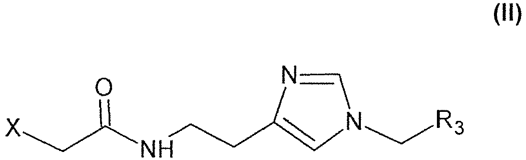 wherein X designates the chelator.

7. Compound V according to one of the previous claims, **characterized in that** compound V is
(i) 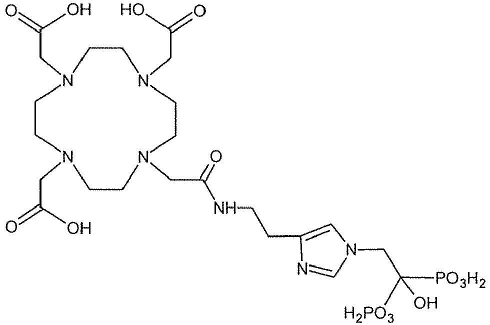 or
(ii) a derivative of (i), wherein the derivative has L₁, L₂, L₃, R₁, R₂, R₃ as defined by (i) and the chelator X is DOTAM.

8. Pharmaceutical consisting of the compound V according to claim 1, 2, 3, 4, 5, 6 or 7, and a metallic isotope M complexed with the compound V.

9. Pharmaceutical according to claim 8, **characterized in that** the metallic isotope M is selected from the group comprising ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ^{99m}Tc, ¹¹¹In, ¹³⁵Sm, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi and ²²⁵Ac.

10. Method for producing a pharmaceutical according to claim 8 or 9, comprising the following steps:
(a) providing a solution S containing the compound V according to any one of claims 1 to 7;
(b) providing a metallic isotope M, such as ⁶⁸Ga(III); and
(c) ligating the metallic isotope M with the compound V to form a complex MV of the metallic isotope M with the compound V in a solution F.

11. Method according to claim 10, **characterized in that**, in step (b), the metallic isotope M is provided in a solution.

12. Method according to claim 10, **characterized in that**, in step (b), a radionuclide generator with a mother nuclide and a metallic isotope M formed via decay of the mother nuclide is provided, and, in step (c), the metallic isotope M is separated from the mother nuclide with the solution S.

13. Use of a compound V according to claim 1, 2, 3, 4, 5, 6 or 7 as a labelling precursor for producing a pharmaceutical.

14. A pharmaceutical according to claim 8 or 9 for use in an imaging method by means of positron emission tomography or single photon emission computer tomography, in particular for imaging of pharmacokinetic processes, or for use for diagnostic imaging by means of magnetic resonance tomography (nuclear magnetic resonance tomography) or optical imaging.

15. Pharmaceutical according to claim 8 or 9 for use in a treatment or therapy method.

16. Pharmaceutical according to claim 8 or 9 for use in a method for treatment of bone diseases and bone tumors.

17. Pharmaceutical for use according to claim 16 for treatment of diseases of unmanifested bone metastases.

18. Pharmaceutical for use according to claim 17, wherein the treatment comprises the accumulation in the tumor cell in order to inhibit farnesyl pyrophosphate synthesis (FPPS).

19. Pharmaceutical according to claim 8 or 9 for use as an additive in artificial bone substance, in bone cement, or in bone implants.

20. Compound V according to claim 1, 2, 3, 4, 5, 6 or 7 in combination with a metallic isotope M, such as gadolinium, for use for producing a pharmaceutical.

21. Compound V according to claim 20, wherein the metallic isotope M is selected from the isotopes as defined in claim 9.

## Revendications

1. Composé V destiné à la complexation d'isotopes métalliques, comprenant un agent chélateur X et un ou plusieurs vecteurs de ciblage conjugués avec l'agent chélateur X, possédant la structure -L₁-R₁-L₂-R₂-L₃-R₃-dans laquelle :
L₁ est choisi parmi le groupe comprenant un groupe amide, un groupe phosphinate, un groupe alkyle, un groupe triazole, un groupe thio-urée, un groupe éthylène, un groupe maléimide, un groupe -(CH₂)ₖ- et un groupe (CH₂CH₂O)ₖ- dans lesquels k est égal à 1, 2, 3, 4, 5, 6, 7, 8, 9 ou 10 ;
L₂ est choisi parmi un groupe -(CH₂)ₘ- et un groupe -(CH₂CH₂O)ₘ- dans lesquels m est égal à 1, 2, 3, 4, 5, 6, 7, 8, 9 ou 10 ; et
L₃ est choisi parmi un groupe -(CH₂)ₙ- et un groupe -(CH₂CH₂O)ₙ- dans lesquels n est égal à 1, 2, 3, 4, 5, 6, 7, 8, 9 ou 10 ;
**caractérisé en ce que**
R₁ représente : 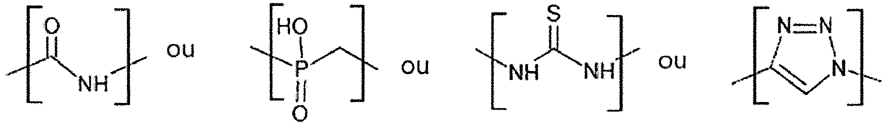
R₂ est choisi parmi le groupe comprenant un groupe faisant office de radical choisi parmi un radical pyrrole, un radical pyridine, un radical pyrimidine, un radical furane, un radical azole, un radical triazole, un radical tetrazole, un radical pyrazole, un radical imidazole, un radical oxazole, un radical oxadiazole, un radical thiophène, un radical thiazole, un radical thiadiazole, un radical azine, un radical oxazine, un radical thiazine, un radical naphtalène, un radical quinoléine, un radical chromène, ou un radical thiochromène ;
et
R₃ représente :

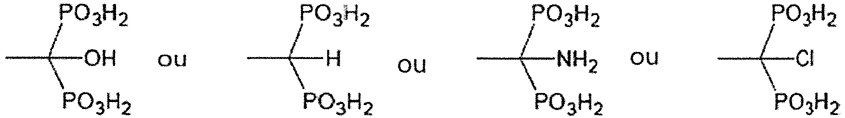

2. Composé V selon la revendication 1, **caractérisé en ce que** l'agent chélateur X est choisi parmi le groupe comprenant du EDTA (éthylènediaminetétraacétate), du EDTMP (acide diéthylènetriamine-penta(méthylène phosphonique)), du DTPA (acide diéthylènetriamine-pentaacétique) et ses dérivés, du DOTA (acide 1,4,7,10-tétraazacyclododécane-1,4,7,10-tétraacétique), du DOTAGA (acide 1,4,7,10-tétramine-triacétique, acide dodéca-1-glutarique), du DOTAM (1,4,7,10-tétrakis(carbamoylméthyl)-1,4,7,10-tétraazacyclododécane) et d'autres dérivés du DOTA, du TRITA (acide tridéca-1,4,7,10-tétraminetétraacétique), du TETA (acide tétradéca-1,4,8,11-tétramine-tétraacétique) et ses dérivés, du NOTA (acide nona-1,4,7-triamine-triacétique) et ses dérivés, comme par exemple le NOTAGA (1,4,7-triazacyclononane, acide 1-glutarique, acide 4,7-acétique), du NOPO (acide 1,4,7-triazacyclononane-1,4-bis[acide méthylène(hydroxyméthyl)phosphinique]-7-[acide méthylène(2-carboxyéthyl)phosphinique]) et ses dérivés, du PEPA (acide pentadéca-1,4,7,10,13-pentaminepentaacétique) et ses dérivés, du HEHA (acide hexadéca-1,4,7,10,13,16-hexamine-hexaacétique) et ses dérivés, du HBED (hydroxybenzyl-éthylène-diamine) et ses dérivés, du DEDPA et ses dérivés tels que le H₂DEDPA (1,2-[{6-(carboxylate)-pyridin-2-yl}méthylamine]éthane), du DFO (déféroxamine) et ses dérivés, du déféripron, du CP256 (bis-[(3-hydroxy-1,6-diméthyl-4-oxo-1,4-dihydro-pyridin-2-ylméthyl)-amide]) de l'acide (4-acétylamino-4-{2-[(3-hydroxy-1,6-diméthyl-4-oxo-1,4-dihydro-pyridin-2-ylméthyl)-carbamoyl]-éthyl}-heptanedioïque) et ses dérivés, tel que le YM103 ; du TRAP (acide triazacyclononane-phosphinique) et ses dérivés, du TEAP (acide tétraazacyclodécane-phosphinique) et ses dérivés, du AAZTA (acide 6-amino-6-méthylperhydro-1,4-diazépine-N,N,N',N'-tétraacétique) et ses dérivés tels que le DATA, et du SarAr (1-N-(4-aminobenzyl)-3,6,10,13,16,19-hexaazabicyclo[6.6.6]-éicosane-1,8-diamine), ainsi que leurs sels.

3. Composé V selon la revendication 1 ou 2, **caractérisé en ce que** le composé V présente une structure répondant à la formule I : 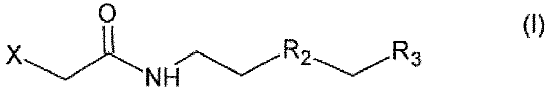
dans laquelle X représente l'agent chélateur ;
ou
**en ce que** le composé V présente une structure répondant à la formule DOTAGA-L₁-R₁-L₂-R₂-L₃-R₃ :

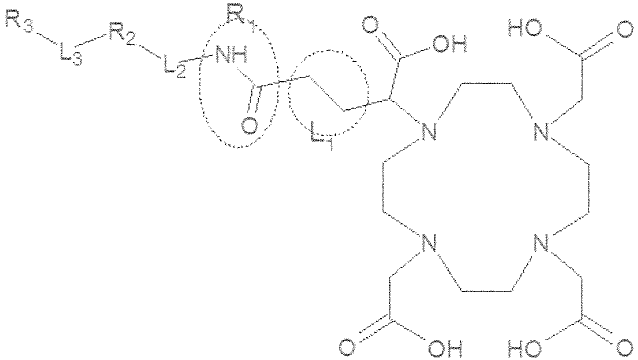

4. Composé V selon la revendication 1, 2 ou 3, **caractérisé en ce que** le composé V présente une des structures suivantes : 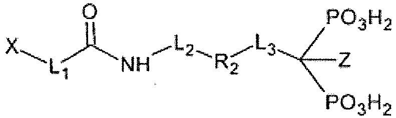 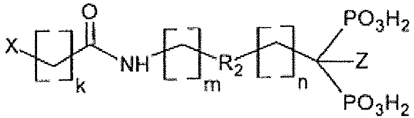 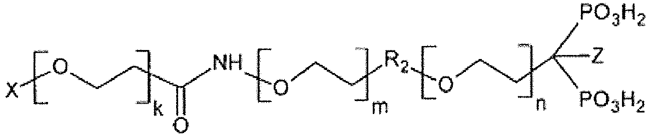 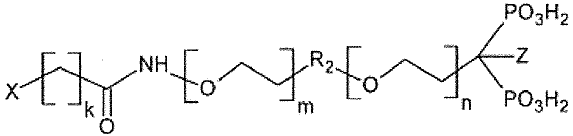 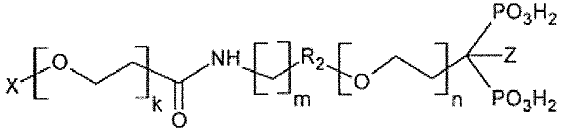 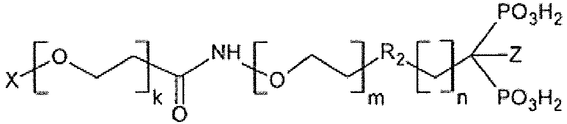 dans lesquelles :
k est égal à 1, 2, 3, 4, 5, 6, 7, 8, 9 ou 10 ;
m est égal à 1, 2, 3, 4, 5, 6, 7, 8, 9 ou 10 ;
n est égal à 1, 2, 3, 4, 5, 6, 7, 8, 9 ou 10 ; et
Z représente un groupe OH, un atome d'hydrogène, un groupe NH₂ ou un atome de chlore.

5. Composé V selon la revendication 1, 2, 3 ou 4, **caractérisé en ce que** R₂ est choisi parmi le groupe comprenant un groupe faisant office de radical choisi parmi :

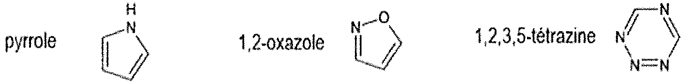

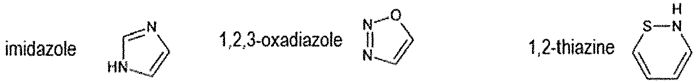

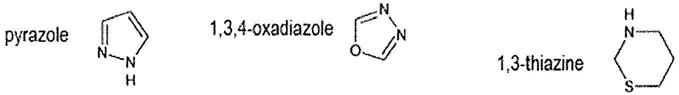

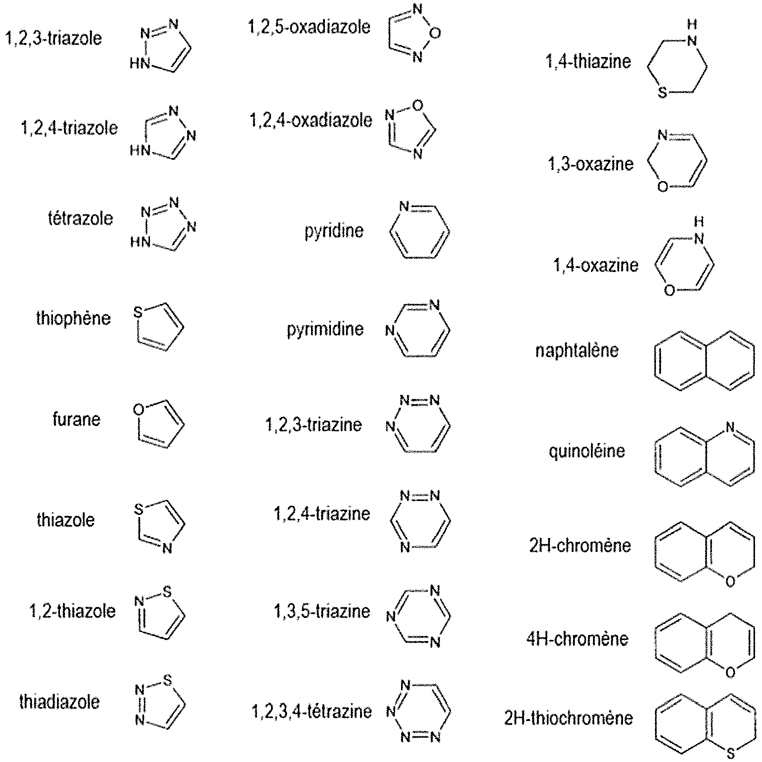

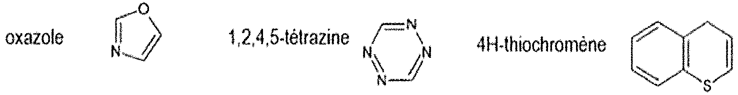

6. Composé V selon l'une quelconque des revendications précédentes 1, 2, 3 ou 4, **caractérisé en ce que** le composé V présente une structure répondant à la formule II : 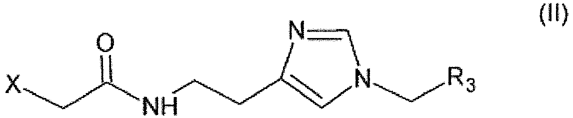 dans laquelle X représente à chaque fois l'agent chélateur.

7. Composé V selon l'une quelconque des revendications précédentes, **caractérisé en ce que** le composé V représente
(i) 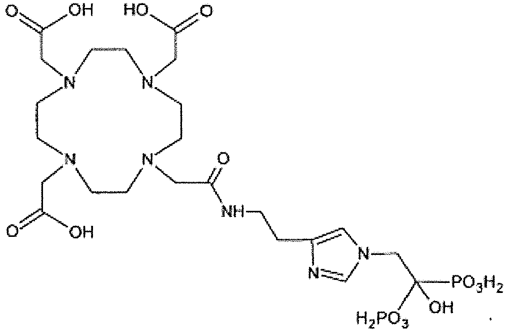 ou
(ii) un dérivé de (i), dans lequel le dérivé présente L₁, L₂, L₃, R₁, R₂, R₃, comme défini sous (i) et l'agent chélateur X représente du DOTAM.

8. Produit pharmaceutique constitué par le composé V selon la revendication 1, 2, 3, 4, 5, 6 ou 7, et par un isotope métallique M complexé avec le composé V.

9. Produit pharmaceutique selon la revendication 8, **caractérisé en ce que** l'isotope métallique M est choisi parmi le groupe comprenant ⁴⁴Sc, ⁴⁷Sc, ⁵⁵Co, ⁶²Cu, ⁶⁴Cu, ⁶⁷Cu, ⁶⁶Ga, ⁶⁷Ga, ⁶⁸Ga, ⁸⁹Zr, ⁸⁶Y, ⁹⁰Y, ⁹⁰Nb, ⁹⁹mTc, ¹¹¹In, ¹³⁵Sm, ¹⁵⁹Gd, ¹⁴⁹Tb, ¹⁶⁰Tb, ¹⁶¹Tb, ¹⁶⁵Er, ¹⁶⁶Dy, ¹⁶⁶Ho, ¹⁷⁵Yb, ¹⁷⁷Lu, ¹⁸⁶Re, ¹⁸⁸Re, ²¹³Bi et ²²⁵Ac.

10. Procédé pour la préparation d'un produit pharmaceutique selon la revendication 8 ou 9, qui comprend les étapes suivantes dans lesquelles :
(a) on procure une solution S qui contient le composé V selon l'une quelconque des revendications 1 à 7 ;
(b) on procure un isotope métallique M, comme par exemple ⁶⁸Ga(III) ; et
(c) on allie l'isotope métallique M avec le composé V pour former un complexe MV de l'isotope métallique M avec le composé V dans une solution F.

11. Procédé selon la revendication 10, **caractérisé en ce qu'**on procure, à l'étape (b), l'isotope métallique M dans une solution.

12. Procédé selon la revendication 10, **caractérisé en ce qu'**on procure, à l'étape (b), un générateur de radionucléide comprenant un nucléide mère et un isotope métallique M que l'on obtient grâce à la décomposition du nucléide mère et, à l'étape (c), on sépare l'isotope métallique M avec la solution S par rapport au nucléide mère.

13. Utilisation d'un composé V selon la revendication 1, 2, 3, 4, 5, 6 ou 7, à titre de précurseur de marquage pour la préparation d'un produit pharmaceutique.

14. Produit pharmaceutique selon la revendication 8 ou 9, pour son utilisation dans un procédé de formation d'image au moyen d'une tomographie par émission de positons ou une tomoscintigraphie par émission monophotonique, en particulier pour la reproduction de processus pharmacocinétiques ou pour son utilisation pour la formation d'une image diagnostique au moyen d'une tomographie par résonance magnétique (tomographie de spin nucléaire) ou pour l'imagerie optique.

15. Produit pharmaceutique selon la revendication 8 ou 9, pour son utilisation dans un procédé de traitement ou de thérapie.

16. Produit pharmaceutique selon la revendication 8 ou 9, pour son utilisation dans un procédé de traitement de pathologies osseuses et de tumeurs osseuses.

17. Produit pharmaceutique pour son utilisation selon la revendication 16, pour le traitement de pathologies visant des métastases osseuses non manifestées.

18. Produit pharmaceutique pour son utilisation selon la revendication 17, dans lequel le traitement comprend l'enrichissement dans la cellule tumorale pour l'inhibition de la farnésyl-pyrophosphate-synthase (FPPS).

19. Produit pharmaceutique selon la revendication 8 ou 9, pour son utilisation comme additif dans une substance osseuse artificielle, dans du ciment d'os ou dans des implants osseux.

20. Composé V selon la revendication 1, 2, 3, 4, 5, 6 ou 7, en liaison avec un isotope métallique M, tel que le gadolinium, pour son utilisation lors de la préparation d'un produit pharmaceutique.

21. Composé V pour son utilisation selon la revendication 20, dans lequel l'isotope métallique M est choisi parmi les isotopes mentionnés à la revendication 9.
